(19) Europäisches Patentamt

European Patent Office

Office européen des brevets

(11) **EP 2 409 643 B1**

(12) **EUROPEAN PATENT SPECIFICATION**

(45) Date of publication and mention
of the grant of the patent:
**08.01.2020 Bulletin 2020/02**

(51) Int Cl.:
***A61B 5/06*** *(2006.01)*     ***A61B 8/08*** *(2006.01)*
***A61M 25/01*** *(2006.01)*

(21) Application number: **10753118.8**

(22) Date of filing: **16.03.2010**

(86) International application number:
**PCT/CN2010/071068**

(87) International publication number:
**WO 2010/105551 (23.09.2010 Gazette 2010/38)**

(54) **HUMAN CAVITY WALL THREE-DIMENSIONAL MEASURE METHOD, INSTRUMENT AND SYSTEM**

VERFAHREN, INSTRUMENT UND SYSTEM ZUR DREIDIMENSIONALEN MESSUNG MENSCHLICHER HOHLRAUMWÄNDE

PROCÉDÉ DE MESURE TRIDIMENSIONNELLE D'UNE PAROI DE CAVITÉ HUMAINE, ET INSTRUMENT ET SYSTÈME AFFÉRENT

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **16.03.2009 CN 200910047713**

(43) Date of publication of application:
**25.01.2012 Bulletin 2012/04**

(73) Proprietor: **Shanghai Microport EP Medtech Co., Ltd.**
**Shanghai 201318 (CN)**

(72) Inventors:
• **LIU, Daozhi**
**Shanghai 201203 (CN)**
• **SUN, Yiyong**
**Shanghai 201203 (CN)**
• **MA, Changsheng**
**Shanghai 201203 (CN)**
• **ZHANG, Guowang**
**Shanghai 201203 (CN)**
• **GUO, Junmin**
**Shanghai 201203 (CN)**
• **CHENG, Huasheng**
**Shanghai 201203 (CN)**

(74) Representative: **Kleine, Hubertus et al**
**Loesenbeck - Specht - Dantz**
**Patent- und Rechtsanwälte**
**Am Zwinger 2**
**33602 Bielefeld (DE)**

(56) References cited:
**EP-A2- 1 321 097**     **EP-A2- 1 760 661**
**WO-A1-90/13259**     **WO-A1-92/03090**
**WO-A1-93/04628**     **WO-A2-2007/061890**
**CN-A- 1 168 625**     **CN-A- 101 243 975**
**US-B1- 6 432 136**

## Description

The present application claims priority right of the Chinese patent application No. 200910047713.2 in the title of "Human Cavity Inner Wall Three-Dimensional Mapping Method, Equipment and System" filed with the Chinese Patent Office on Mar. 16, 2009.

## Technical Field

The present invention relates to a human cavity inner wall three-dimensional mapping method, equipment and system, which is mainly used for visualized navigation of an intracardiac electrophysiology catheter, construction and registration of a heart cavity inner wall three-dimensional electroanatomic image, and synchronous processing of a plurality of channels of electrophysiological signals, thereby assisting a clinic doctor to diagnose arrhythmia and treat it through an operation.

## Background Art

Atrial fibrillation is one of the most common diseases of arrhythmia in the world, and it often causes a patient to suffer serious complications such as stroke and so on, seriously influencing life quality of the patient. With the broad development and gradual maturation of radiofrequency catheter ablation technology on arrhythmia diseases such as paroxysmal supraventicuar tachycardia, idiopathic ventricular tachycardia, atrial flutter and so on, more and more clinic doctors start to divert the radiofrequency catheter ablation to the treatment of atrial fibrillation. It has been currently proved in the medical world that atrial fibrillation, particularly the paroxysmal atrial fibrillation, can be effectively treated by applying radiofrequency catheter ablation to electro-excitability focal targets such as atrial isthmus, rear wall and so on, and particularly by performing an annular radiofrequency ablation conduction isolation at openings of a few pulmonary veins of the atrial. Since the pathogenic mechanism of atrial fibrillation is complicated, and there are many myocardial tissues that need to be ablated, the conventional instruments for treating common arrhythmia diseases such as the supraventicuar tachycardia, i.e., multi-channel physiological recorder and X-ray fluoroscopy instrument, have been unable to meet the demand of atrial fibrillation ablation operations. The main limitations lie in that a lot of positions need to be ablated, which causes the operation duration to be very long, and the X-ray instrument needs to be frequently started in order to observe the position of the catheter, thus the patient and the doctor suffer excessive X-ray damage, and this is hard to be accepted in clinic. In addition, although the conventional multi-channel physiological recorder can provide plenty of electrophysiological signals, it is unable to provide mapping of heart cavity mechanical structure, so it is unable to meet the need of catheter navigation in clinic operation. Thus, it is very important to the treatment of diseases such as atrial fibrillation and so on to create a three-dimensional electroanatomical mapping system capable of reducing reliance on X-ray fluoroscopy, implementing real-time three-dimensional catheter navigation, recreating a three-dimensional electroanatomical structure of a heart cavity while displaying necessary physiological signals.

The American patent application US2004/0254437 in the title of "METHOD AND APPARATUS FOR CATHETER NAVIGATION AND LOCATION AND MAPPING THE HEART", the patent CN1Q1147676A in the title of "Endocardium three-dimension navigation system and navigation method ", the patent CN200970234Y in the title of "Endocardium three dimension navigation system ", and the patent CN201033076Y in the title of "Heart pipe three-dimensional marking and measuring system " relate to a cardiac catheter three-dimensional navigation system, whose principle is as follows: placing three pairs of spatially orthogonal excitation electrodes on the body surface of a patient, typically on the chest-the back, the left axilla-the right axilla, the back of the neck-inner side of the left thigh of the patient; applying power to the three pairs of excitation electrodes to form a three-dimensional electric field; collecting, by a catheter electrode, an electric field signal at the position where the catheter is located in the three-dimensional electric field; calculating position information of the catheter based on the electric field signal, thereby implementing the catheter navigation and the pattern recreation of heart cavity mechanical structure. However, this navigation system structure based on the three-dimensional orthogonal electric field is complex, the position data of the catheter is not directly taken from the spatial positioning data but is inversely deduced by using complicated empirical algorithms, by means of which, a three-dimensional electroanatomical structure of a heart cavity is created. On one hand, this system is easily affected by the electric conduction feature of muscle tissue and breath of a patient, resulting in distortion of the catheter position and the created cavity structure. On the other hand, this system has a very high requirement on the gain consistency of an amplifying and collecting circuit that collects electrical signals of respective channel electrodes and the catheter, and signals will be inevitably distorted if the gains are not consistent. However, it is hard to ensure consistency of gains for respective channels in practical application since circuits are easily affected by changes in temperature and own characteristics of electronic elements are not consistent, and even by means of complicated compensation circuits and algorithms, it is still unable to ensure consistency of gains for respective channels. Further, the system vulnerable by the influence of other external interference electric fields will also cause the distortion of the heart cavity structure. Therefore, the mapping systems based on the three-dimensional orthogonal electric field in the above mentioned patents generally have a complicated structure and algorithms, are difficult to adapt to special cases of different patients, and always have a certain distortion in the heart cavity mechanical structure and catheter position.

It is known that the spatial position of a target can be tracked by using a field positioning equipment. This technology has started to be applied in various fields including mechanical instrument field. The U.S. patent US6,385,482 discloses a method of measuring the position of a target device in certain space, the U.S.patent US6,553,326 discloses a method of measuring a change in magnetic location or magnetic orientation, and the U.S.patent US6,625,563 discloses a system of measuring the position, orientation, and system gain factor of a detector. In addition, the patent CN1168625A by BEN-HAIM S et al discloses a medical diagnosis, treatment and imaging system, whose core content relates to a medical probe whose position can be detected and adjusted, and having functions of auxiliary detecting, imaging and/or treatment. This patent describes a location tracking system based on magnetic field positioning, comprising three non-overlapped magnetic field generating device that track the position of an intrusive medical instrument based on an Alternating Current (AC) magnetic field and obtain three position coordinates and three orientation coordinates for one point on this instrument. This system emphasizes acquiring six-dimensional coordinate information of one spatial point by using three sensors.

The magnetic field position tracking system described in these patents still has the following technical problems to be solved in practical application:

1) There are three axially orthogonal magnetic field sensors near the distal end portion of the intrusive instrument in the system, and coordinates of one point on the catheter are mapped through the three sensors. Since deviations in relative positions and angles among the three orthogonal sensors greatly influence positioning accuracy, and it needs a complicated system structure and algorithm to calibrate and maintain the accuracy of the relative positions and angles, three sensors are used for acquiring coordinates of one spatial point, resulting in high cost and huge volume.

2) The catheter structure and method described in the system can only obtain spatial coordinates of one point on the catheter, so it is only able to simulate and display the fixed form of the catheter' distal end portion. This is unable to provide a clinic doctor with necessary display of a long variable bending portion of the catheter. Since the heart cavity structure is complicated, the doctor needs to frequently manipulate and move the catheter so that the catheter's distal end portion can arrive at the desired position. The doctor must know the current bending shape of the catheter when manipulating and moving the catheter. It is unable to provide display of the bending shape if only the distal end portion is displayed, and it still needs to determine the bending shape and orientation of the catheter by means of X-ray fluoroscopy, thereby increasing radiation damages to the patient and the doctor.

Except for these technical problems to be solved, the existing three-dimensional mapping system or device created based on the electro-magnetic field position tracking principle in the above patents still have the following issues that need to be improved:

1) This type of equipment or system generally can only display a limited few channels of intracardiac electrocardiogram (ECG) signals. However, during the operation of arrhythmia such as atrial fibrillation and so on, at least ten channels of annular pulmonary vein signals and multiple channels of coronary sinus physiological signals need to be additionally added in addition to a few channels of Intracardiac ECG (IECG) signals of the catheter needing to be navigated. This system cannot satisfy this demand, and must depend on the cooperation of multi-channel electrophysiological recorders of other manufacturers. As a result, the system integration degree is not high, the connection between equipments is complicated, and additional communication protocols and hardware connections are required, which increase additional interferences and risk in safety, and occupy more space in an operation room. Meanwhile, more doctors need to be arranged to watch several systems. Operation data are individually stored in different systems and cannot be integrally stored, and the desired various data cannot be synchronously reproduced after the operation.

2) This equipment or system generally emphasizes that six-dimensional coordinates for each point need to be obtained, and each point needs three sensors in order to realize the six-dimensional coordinates, resulting in a complicated catheter structure and high cost.

3) When this equipment or system is used for three-dimensional mapping, it generally can only display a fixed shape of the distal-tip portion of the catheter, and cannot display the bending shape, whereas the necessity of the bending shape of the catheter in an operation has been described in the above.

Patent publications EP1321097 (A2), WO2007/061890 (A2) and EP1760661 (A2) are also relevant for understanding the background of the present invention.

## Summary of the Invention

The purpose of conducting a deep research on the development and usage of the three-dimensional mapping system introduced in the Background Art in the present invention is to provide an improved three-dimensional mapping method and a corresponding equipment and system. The technical solution of the present invention employs a new type of catheter positioning technology combined with plenty of electrophysiological signals, with a simple structure, low cost, plenty of functions, high integrity, high implementability and safety.

The three-dimensional mapping system generally comprises one or more target devices, which are generally intrusive medical instruments. How to acquire spatial position information of a specific point on the target device in real time is one of the key techniques in the present system for implementing three-dimensional navigation of the target device and creating a three-dimensional image of a cavity inner wall by moving the target device inside the cavity of a human body, such as the heart cavity. A preferred applicable heart three-dimensional mapping system in technical solutions of the present invention will be specifically exemplified thereafter.

In an application environment of performing a three-dimensional mapping on a heart, the target device may be a cardiac catheter. In order to create a three-dimensional image of the structure of the heart cavity inner wall, the precondition is to acquire the spatial position information of a part where the catheter touches the heart cavity inner wall, typically, the spatial three-dimensional coordinates of one point on the distal end of the catheter. The movement spatial track data of the catheter is acquired by moving the catheter distal end on each feature part on the heart cavity inner wall so as to map spatial position data of a plurality of points on the heart cavity inner wall, thereby creating a three-dimensional image model of the heart cavity inner wall by using a software imaging technique.

If it further needs to carry out three-dimensional image real-time navigation of the distal end portion of the catheter, at least two angular orientation coordinates of the catheter distal end need to be further acquired. These angular orientation coordinates may be determined by using quaternion algorithm, Euler angles or other suitable methods. For example, the sensor coil described in the present invention may be regarded as a rigid body in a three-dimensional geometrical space. The angle parameters of the movement of the sensor coil include: a self rotation angle j indicating axial rotation of the sensor coil about the center, and a nutation angle $\theta$ and a procession angle $\psi$ indicating vertical and horizontal rotation of the sensor coil. Preferably, the central axial direction of the coil of the magnetic field sensor in the catheter is parallel with the axial direction of the cavity of the catheter in the present invention, and in order to simplify the process of acquiring and processing coordinate data, the distal end portion of the catheter in the present invention is designed as a cylinder or in other shape that is symmetrical with respect to the axis. Accordingly, during the process of acquiring and processing the coordinate data, the distal end portion of the catheter can be regarded as a cylinder. In the precondition of such setting, it may be regarded that the axial rotation of the catheter distal end has no influence on the signal collection, the contact between the catheter distal end and the heart cavity inner wall, and the transmission of treatment signals, and has no influence on the creation of the three-dimensional image of the heart cavity inner wall, thus it is unnecessary to consider the influence by the change in self-rotation angle coordinate j, and only the nutation angle $\theta$ and the procession angle $\psi$ need to be acquired and processed. The data of five-dimensional position and orientation coordinates or five-dimensional coordinates in the present invention refer to the three-dimensional position coordinates and the nutation angle $\theta$ and the procession angle $\psi$ unless it is specifically specified,.

The purpose of a first aspect of the present invention is to provide a three-dimensional mapping method suitable for a human cavity inner wall, for determining a position and orientation of a target device with respect to a reference system, said method comprises the steps of:

a. generating a plurality of distinguishable fields by using a drive signal; generally the fields in the technical solution of the present invention are AC magnetic fields. Preferably, these AC magnetic fields are continuous magnetic fields.
b. detecting the distinguishable fields by at least one sensor preset in said target device so as to generate a sensor signal; generally, said sensor in the technical solution of the present invention comprises one metallic coil, and said one metallic coil is a magnetic field sensor.
c. detecting an output of the sensor signal to acquire data of five-dimensional position and orientation coordinates of a point on said target device where the sensor is located.

In the technical solution of the present invention, the plurality of distinguishable fields respectively have different frequencies or different phases, or have both different frequencies and different phases. Said drive signal used for generating the plurality of distinguishable fields can be cross-correlated to the sensor signal. When the frequencies of the plurality of distinguishable fields are integer multiples of a given frequency, the duration of said cross-correlation is a result obtained by dividing the least common multiple of said integer multiples by said given frequency. The result of the cross-correlation may be used for calculating respective contributions of the plurality of distinguishable fields to the signals generated by said sensors.

In order to describe the contour or shape of the human cavity inner wall, such as the shape of the heart cavity inner wall, this method may further comprise the steps of:

d. acquiring again the data of five-dimensional position and orientation coordinates of the point on said target device where the sensor is located by moving said target device along the human cavity inner wall;

e. repeating steps b to d to acquire a plurality of groups of the data of five-dimensional position and orientation coordinates;

f. creating a three-dimensional image model using the data of three-dimensional position coordinates and/or data of two orientation coordinates in the acquired plurality of groups of the data of five-dimensional position and orientation coordinates.

In practical, application, it generally needs to move the target device for several times in the cavity of the human body. For example, a doctor needs to control the catheter that has entered into a heart to move accurately and perform a corresponding manipulation at a specific position during the operation, such as the ablation of a part of a tissue by using a radiofrequency energy signal. In this case, even if a three-dimensional image model of the human cavity inner wall has been created, if only the position of one point or one portion on the target device with respect to the reference system can be determined, it is still difficult for the manipulator to grasp the whole state of the target device in the cavity, so that it is difficult to accurately and effectively control the movement of the target device in the cavity.

In the technical solution of the present invention, the above mentioned technical problem can be overcome by arranging more than one sensor on the target device. Generally, the target device may be preset with more than one sensor, and these sensors are mutually spaced apart by an equal or unequal distance. Preferably, the target device may be preset with two sensors that are mutually spaced apart by a certain distance. In the case where the target device is a cardiac catheter, the two sensors may be arranged as such that one is close to the distal end portion of the target device, and the other is spaced apart from the distal end of the target device by a certain distance. In this case, the present invention may implement the following steps by using the more than one sensor:

g. acquiring the data of five-dimensional position and orientation coordinates of points on the target device where the more than one sensor is located by using the data respectively measured by the more than one sensor preset in said target device; and

h. acquiring a partial or whole three-dimensional image model of the target device through simulation by using the data of three-dimensional position coordinates and/or data of two orientation coordinates in the acquired data of five-dimensional position and orientation coordinates of the points on the target device where the more than one sensor is located.

In a preferred implementation, the present invention may further record an electrical signal of a current position by means of an electrode preset in the target device. For example, when the target device is on the surface of the heart cavity inner wall, it is able to record the electrical signal of the heart cavity inner wall. It is possible to form a map indicating the propagation situation of the electrical signals via the tissue of the heart cavity inner wall by combining the collected plurality of electrical signals of said heart cavity inner wall with the correspondingly collected data of five-dimensional position and orientation coordinates. Thus, it is possible to vividly display the transmission situation of the electrical signals on the surface of the tissue of the heart cavity inner wall in an image.

The purpose of a second aspect of the present invention is to provide a field positioning equipment for determining a position and an orientation of a target device with respect to a reference system. Generally, the target device may be an intrusive medical instrument, for example, a cardiac catheter. The field positioning equipment comprises:

at least one field generating device, which, under the excitation of a drive signal, generates recognizable and distinguishable fields; generally but not limited, said field generating device may contain a plurality of metallic coils that respectively generate a plurality of distinguishable fields under the excitation of the drive signal.

at least one field sensor located in said target device for detecting the distinguishable fields to generate a sensor signal; the plurality of distinguishable fields generated by the field generating device respectively have different frequencies or different phases, or have both different frequencies and different phases. Preferably, the frequencies of said plurality of distinguishable fields are integer multiples of a given frequency. Generally but not limited, each of said sensors is comprised of a metallic coil. When said target device is an intrusive medical instrument, for example, a cardiac catheter, said field sensor is located in the distal end portion of the cardiac catheter. In the technical solution of the present invention, said fields are generally AC magnetic fields, and preferably, said AC magnetic fields are continuous magnetic fields.

a positioning signal processing device that may have a plurality of interfaces respectively connected to the field generating device and the field sensor, for transmitting the drive signal to said field generating device and detecting an output of said field sensor signal, thereby acquiring five-dimensional position and orientation data of a point on said target device where the sensor is located. More specifically, said positioning signal processing device may cross-correlate said drive signal to said sensor signal, wherein the duration of said cross-correlation is a result obtained by dividing the least common multiple of the integer multiples by said given frequency. Said positioning signal processing device uses the result of said cross-correlation to calculate respective contributions or influence factors of said plurality of distinguishable fields to the signal generated by said sensor. Preferably, said equipment may comprise a three-dimensional image simulating device for processing said data of five-dimensional position and orientation coordinates and creating a three-dimensional image model based on data of three-dimensional position coordinates and/or data of two orientation coordinates in said data of five-dimensional position and orientation coordinates.

Generally, the field positioning equipment provided in the present invention can acquire three position coordinates and two orientation coordinates of a point on the target device by merely using one field sensor, i.e., one metallic coil. But this does not exclude the case that the sensor of the present invention may contain a plurality of coils. Since the field positioning system of the present invention merely needs one metallic coil, the positioning part may have a smaller volume, a simpler structure, a lower cost, and higher implementability.

In one preferred implementation, the field positioning equipment described in the present invention can not only be used for determining the position and the orientation of the target device with respect to the coordinate system of the system, but also enable the target device to be moved and positioned more effectively. This solution is implemented by placing more than one field sensor at different positions of the target device, for example, the distal end portion of a cardiac catheter. Generally, the more than one sensor is mutually spaced apart by an equal or unequal distance. For example, when the target device is a cardiac catheter, more than one field sensor may be placed on the catheter by a certain distance. Preferably, the target device comprises two field sensors that are mutually spaced apart by a certain distance. By using such an arrangement of the sensors, it is possible to acquire the data of five-dimensional position and orientation coordinates of more than one point on the target device. Since the catheter specification fit for the equipment, such as diameter and material of the catheter, the number of sensors preset in the catheter, the distance between sensors when there are a plurality of the sensors, and the distance between the catheter distal end and the sensors, are known, and on the other hand, the specifications and positions of the other functional device, such as preset electrode, are also known, the field positioning equipment of the present invention can thereby simulate a partial three-dimensional image of the portion near the tip of the catheter by using a mathematical method based on these data. This measure of simulating a partial three-dimensional image of the catheter by collecting five-dimensional data of a plurality of points on the catheter may draw support from a conventional mathematical simulation method. Since the mathematical simulation method is a matured prior art and not a key point of the present invention, it is not described in detail herein.

The simulated three-dimensional image of a partial catheter may vividly guide a doctor to manipulate the catheter to move toward the target on the heart cavity inner wall so that the operation is quicker and more efficient. For example, in an electrophysiological radiofrequency ablation operation, the doctor needs to manipulate the catheter to move a plurality of times inside the heart cavity to acquire necessary intracardiac ECG signals and apply energy signals to the target. In order to make the catheter to be in well contact with the wall, the catheter itself needs to be formed in a certain arc to ensure the necessary pressure force. When moving the catheter toward the target, the catheter needs be manipulated to make its bending arc to be bent toward the desired direction. If the bending shape of the catheter cannot be displayed, the doctor usually makes his/her judgment based on experiences and memories. In most cases, the doctor needs the help of repeating X -ray fluoroscopy to make an observation and conduct unnecessary test movements. This will prolong the operation time, and increase damages of X-ray to the patient and doctor. The display of simulated bending shape of the catheter provided in the preferred solution of the present invention can effectively help the manipulator to move and position the catheter. Since the simulated bending shape of the catheter is very vivid, the manipulator will no longer need the help of X -ray fluoroscopy to make the observation and perform unnecessary test movements, and thus all above disadvantages may be avoided.

In another preferred solution, the target device of the field positioning equipment provided in the present invention further comprises an electrical signal sensor for recording an electrical signal of the position where the target device is located. For example, when the target device is located on the surface of the heart cavity inner wall, it is able to record electrical signals of the heart cavity inner wall. The positioning signal processing device may form a map indicating the propagation situation of the electrical signals via the tissue of the heart cavity inner wall by combining the collected plurality of electrical signals of the heart cavity inner wall with the correspondingly collected data of five-dimensional position and orientation coordinates and processing them. Thus, it is possible to vividly display the transmission situation of the electrical signals on the tissue of the heart cavity inner wall in an image.

Complementarily, the field in any one of the field positioning equipments mentioned in the present invention may be an AC magnetic field, typically a continuous sine-wave AC magnetic field. The field in any one of the field positioning equipments mentioned in the present invention may also be a DC pulse magnetic field, typically a periodically-changed DC pulse magnetic field.

Each field sensor in any one of the field positioning equipments mentioned in the present invention is comprised of a metallic coil. One preferred embodiment of the arrangement mode of the sensor in the catheter is that the central axis of the sensor coincides with the longitudinal central axis of the cavity of the catheter.

The purpose of a third aspect of the present invention is to provide a three-dimensional electroanotomical mapping system used in a human cavity. Said system may comprise any one of the previously described field positioning equipments, and may be added with various working devices or functional devices; and it may be cooperated with the field positioning equipment to form one system. Said working devices or functional devices are generally located in the target device, and may produce a function or implement a certain function for a position where the target device is located in response to a control signal.

Generally, said working device or functional device may comprise: a tabular device having a hole on the target device for delivering a liquid to the place where the target device is located; an electrode for supplying a power to a substance that touches the electrode to ablate a portion of the substance that touches the electrode; a temperature sensor for measuring a temperature of a place where the target device is located so as to control the release of ablation energy based on the temperature signal; a pressure sensor for measuring how good a contact between the target device and the tissue of the human cavity inner wall is. The person skilled in the art should understand that these devices are merely used for exemplification not for limitation, and he/she may select various functional devices according to the requirement.

The system of the present invention may further comprise a body surface reference positioning electrode mounted on the surface part of a human body. The data of three-dimensional spatial coordinates and three orientation coordinates of one point on the body surface reference positioning electrode is determined by at least two micro electromagnetic fields preset therein so as to provide one reference point, which is still with respect to the surface part of the human body. The system may further selectively comprise an electrical signal reference means for detecting an electrical signal of a specific position in the human cavity. Generally, the electrical signal reference means is placed in the coronary sinus part during the measurement. The electrical signal measured by the electrical signal reference means may be regarded as a reference electrical signal, which is referenced by or compared with the electrical signal of the human cavity inner wall synchronously measured by the electrode in the aforesaid target device.

Preferably, the system of the present invention may comprise an electrical signal processing device for processing electrical signals of a plurality of points on the human cavity inner wall acquired by the electrode such that these electrical signals are combined with the data of five-dimensional position and orientation coordinates of the plurality of points collected by the positioning signal processing device to form a map indicating the propagation situation of the electrical signals via the tissue of the human cavity inner wall.

In addition, the system provided in the present invention may further comprise: a workstation computer for reading data of the positioning signal processing device and the electrical signal processing device, outputting them in a corresponding data output device, and calculating and processing these data; and a display device for visually displaying the data collected in the system and the three-dimensional image model of the human cavity inner wall and/or the target device.

**Description of the Drawings**

The other features, objectives and advantages of the present invention will become more apparent by reading the following detailed description on the non-limited embodiments with reference to the drawings.

The same or similar reference numerals represent the same or corresponding parts in the drawings.

FIG. 1 is a flow chart of a non-limited implementation of a method of performing a three-dimensional mapping in a human cavity with a field positioning equipment provided according to the first aspect of the present invention;

FIG. 2 is a flow chart of detailed steps of navigation of a target device in a human cavity according to one implementation of the present invention;

FIG. 3 is a schematic flow diagram according to a preferred implementation of the present invention;

FIG. 4 is a schematic system diagram of the system of the field positioning equipment provided according to one particular implementation of the present invention;

FIG. 5 is a schematic system diagram of the system of the field positioning equipment provided according to another preferred implementation of the present invention;

FIG. 6 is a schematic diagram of the three-dimensional human cavity electroanatomical mapping system provided according to one particular implementation of the present invention;

FIG. 7 is a schematic diagram of two orientation coordinates in the five-dimensional position and orientation coordinates according to the present invention;

FIG. 8 is a sectional view of a distal end portion of a cardiac catheter according to one particular implementation of the present invention;

FIG. 9 is a sectional view of a distal end portion of a cardiac catheter according to one preferred implementation of the present invention;

FIG. 10 is a sectional view of a distal end portion of a cardiac catheter according to another preferred implementation of the present invention;

FIG. 11 is a schematic diagram of a partial three-dimensional image model of a target device simulated by using the method provided according to one particular implementation of the present invention, the target device is preset with two field sensors; and

FIG. 12 is a schematic diagram of a partial three-dimensional image model of a target device simulated by using the method provided according to one particular implementation of the present invention, the target device is preset with three field sensors.

**Detailed Description of Embodiments**

The technical solution of the present invention will be described in more detail with reference to the following detailed implementations that are merely for exemplification and not construed to limit the present invention. The scope of protection is defined solely by the appended claims.

Based on the previously described technical solution in the Summary of the Invention, this implementation provides a field positioning equipment based on a field positioning technology to implement a method of performing human cavity three-dimensional mapping with the field positioning equipment according to the first aspect of the present invention.

The field positioning equipment provided in this implementation mainly comprises: a field generating device; a field sensor; and a corresponding positioning signal processing device. The detailed process of implementing the method according to the first aspect of the present invention will be described below.

In the field positioning equipment of this implementation, the field generating device is an AC magnetic field generating device for transmitting an AC magnetic field to a space; the field sensor is a five-dimensional magnetic field sensor formed of a coil for inducing the AC magnetic field to generate a voltage/current signal. The field generating device and the field sensor are respectively connected to the positioning signal processing device. When the positioning signal processing device transmits a cyclic control excitation signal, i.e., a drive signal, to the magnetic field generating device, the magnetic field generating device may transmit the AC magnetic field to a space. Generally, the field generating device may be placed below a support table, such as an operation table, so that the transmitted magnetic field can effectively cover the space above the table, and the cavity, such as the heart cavity of a patient that needs to be detected, can be covered by the magnetic field transmitted by the magnetic field generating device when the patient lies down on the support table. In order to achieve the detection purpose, some certain intrusive measures are needed to allow the field sensor to enter into the human cavity. For example, a catheter is used to enter into the heart cavity through a blood vessel of a human body, with the field sensor being preset in the distal end portion of the catheter.

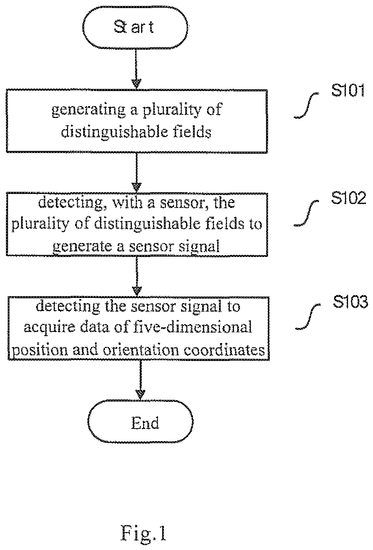

FIG. 1 is a flow chart of a non-limited implementation of a method of performing a three-dimensional mapping in a human cavity with the field positioning equipment provided according to the first aspect of the present invention. Taking the three-dimensional detection in the heart cavity as an example, it is assumed that a patient has lied down on the operation table, and a target device, i.e., the distal end portion of the catheter containing the field sensor has entered into the heart cavity of the patient. The detailed steps of the method mentioned in this implementation are described below with reference to FIG. 1.

At step S101, the positioning signal processing device transmits a cyclic control excitation signal to the magnetic field generating device, so that the magnetic field generating device driven by this drive signal transmits distinguishable AC magnetic fields to the surrounding space including an area above the operation table where the heart of the patient exists.

At step S102, the sensor preset in the distal end portion of the catheter that has been positioned in the heart cavity of the patient and the AC magnetic fields may generate electromagnetic induction so as to generate a voltage/current signal.

At step S103, the voltage/current signal is transferred to the positioning signal processing device through the connection between the magnetic field sensor and the positioning signal processing device. In this implementation, the positioning signal processing device can perform synchronous correlation processing on aforesaid control excitation signal and the voltage/current signal induced by the sensor so as to calculate five-dimensional coordinates including three-dimensional spatial coordinates and two orientation coordinates of the point in the distal end portion of the catheter where the sensor is located.

By using this method, it is possible to obtain the position and orientation information of the distal end portion of the catheter in the cavity of the patient, wherein the position information is used for representing the position of the distal end portion of the catheter (actually it is the place in the catheter distal end where the sensor coil is located, and more specifically, it can be the center of the coil) in the three-dimensional space, and the orientation information is used for marking the orientation of the distal end portion of the catheter (actually it is the sensor coil in the distal end portion of the catheter) in the three-dimensional space.

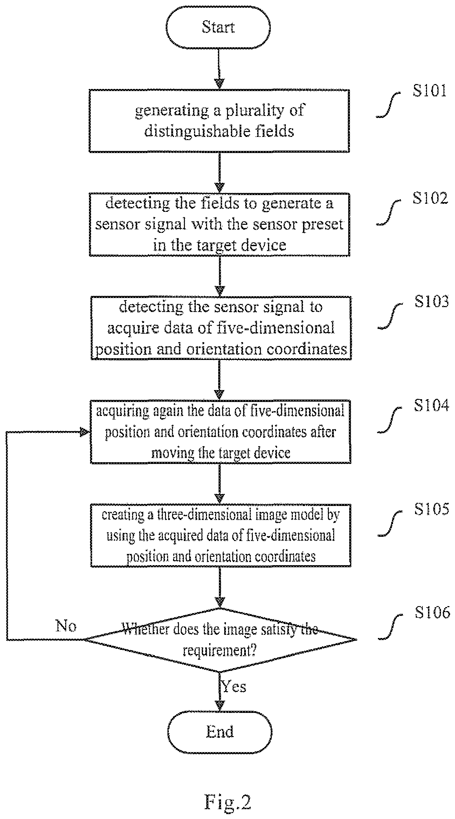

In order to implement navigation of the catheter distal end in the heart cavity, this implementation further provides the following method, referring to FIG. 2 for the detailed steps.

At step S104, the target device, i.e., the cardiac catheter is moved along the heart cavity inner wall in such a way that the distal end portion of the cardiac catheter is moved along the heart cavity inner wall with the distal end portion touching it to obtain again the five-dimensional coordinates of the point where the sensor is located on the target device. In the practical usage, in order to determine whether the distal end portion of the cardiac catheter touches the heart cavity inner wall, an manipulator can usually make judgments by the following measures: monitoring an electrical signal by using an electrode preset in the distal end portion of the catheter (monitoring the ECG in this implementation), and if the distal end portion of the catheter touches the heart inner wall, the magnitude of the signal monitored by the electrode is relatively big; or, the manipulator may also determine whether the distal end portion of the catheter touches the heart cavity inner wall based on the returned hand feeling of the catheter during the manipulation; in addition, the manipulator may also make this judgment with assistance of an X-ray image during the manipulation, or judge whether the distal end portion of the cardiac catheter touches the inner wall by presetting a pressure sensor on the distal end portion of the catheter and making the judgment based on the fed back pressure signal. The aforesaid measures may be used in combination or separately.

At step S105, a three-dimensional image model of the heart cavity inner wall is created by using the obtained data of five-dimensional coordinates. Generally, the model of the cavity inner wall can be simulated by merely using the three-dimensional spatial coordinates in the data of five-dimensional coordinates. In the practical application, however, since the sensor is preset in the cardiac catheter, there is in fact always a certain distance between the place where the cardiac catheter is mounted and the place where the cardiac catheter touches the inner wall. In order to simulate the human cavity inner wall more accurately, each group of obtained data of position coordinates needs to be calibrated for the offset amount before being used to simulate the cavity inner wall so as to remove the error caused by the distance between the place where the sensor is located and the cardiac catheter tip. In this case, all the data of five-dimensional coordinates, i.e., the three-dimensional coordinates and the two orientation coordinates, need to be used for calculating the offset amount to be calibrated. Specifically, in the case where the three-dimensional coordinates of the point in the cardiac catheter where the sensor is located have been obtained, since the distance between the sensor and the cardiac catheter distal end is known, and their relative position is fixed and invariable, the three-dimensional coordinates of one point of the catheter distal end can be calculated by using a mathematical method based on the current data of orientation coordinates of the cardiac catheter, i.e., the two orientation coordinates. The three-dimensional coordinates can be used for simulating the three-dimensional image model of the cavity inner wall. In some special cases, after recording a plurality of groups of position coordinates of specific positions of the human cavity inner wall, it is also possible to obtain the model of the human cavity inner wall through simulation by using the mathematical method based on a corresponding anatomical structure.

At step S106, after forming the three-dimensional image of the human cavity inner wall through simulation, a doctor may then judge whether this image satisfies the requirement or not, for example, whether it can accurately indicate the structure feature of the human cavity inner wall. If this image does not satisfy the requirement, return to the step S104 to repeat the flow of creating the three-dimensional image of the cavity inner wall, and increase sampling points, so that the image is more detailed and vivid. If the doctor thinks that the image satisfies the requirement, the creation of the three-dimensional image can be ended. In fact, this mode allows the target device to move continuously on the human cavity inner wall and record the data, and then feedback continuously its five-dimensional position and orientation data for creating the three-dimensional image of the human cavity inner wall.

During the process of continuously moving the catheter distal end along the heat inner wall, record the continuously varied five-dimensional coordinates during the moving for multiple times. Generally, intensive point data can be acquired by continuous recording, which can create a more vivid three-dimensional image. However, in this mode, the amount of data to be collected is large, and accordingly, the amount of the calculation conducted by the positioning signal processing device is also large, which may exceed its calculation load. Thus, in consideration of saving calculation resources, the frequency of collecting data may be appropriately reduced, i.e., times of recording the five-dimensional coordinates per unit time are reduced. Or, the doctor who performs the operation may also judge, based on his/her experience, how many positions of the coordinates need to be acquired, or which position coordinates that are related to specific positions of the human cavity need to be acquired. Taking the heart cavity as an example, the doctor may select the part that is specific based on the ECG signal, or the part that is structurally specific in anatomy, as the part whose coordinates need to be acquired.

The created three-dimensional image can be used for vividly positioning and navigating the target device, which is a cardiac catheter embedded into a human body in this implementation. Generally, the positioning signal processing device provided in the present invention is attached with a display device, in which both the three-dimensional image of the human cavity inner wall and the distal end portion of the cardiac catheter can be vividly displayed, and when the distal end portion of the cardiac catheter is moved in real time in the human cavity, their relative positions can also be synchronously simulated and displayed. The manipulator can easily determine the position where the catheter is located based on the displayed image, and controls movement of the catheter with an external control device, such as a control handle connected to the catheter.

In the basic solution of this implementation, the target device that is intrusively imbedded into the human cavity only comprises one sensor, or namely one coil. For example, only one sensor coil is preset in the distal end portion of the cardiac catheter. In this case, there is only one position point, i.e., the position where the sensor coil is located can be used for determining the position of one point on the target device. Accordingly, in the above three-dimensional image navigation mode, the point or symbol represented by only one such sensor or coil being used for displaying the position of the target device is visually displayed, and it cannot reflect the detailed form of the target device. Taking the cardiac catheter mentioned in this implementation as an example, this catheter is in an elongated cylindrical shape, and a segment thereof close to the distal end portion can be controlled by a remote handle, and bent or straightened in a certain radian range. After the catheter has entered into the heart cavity, its distal end portion is generally kept in a bending shape of a certain radian so that its tip can better abut against or hook up the heat inner wall. If only one such sensor is used, it will be unable to display the bending form of the catheter. In the practical application, however, the doctor may not effectively operate on the target device because he/she does not know the detailed form of the target device. For example, in the case where the cardiac catheter is inside the heart cavity, even if the position where the distal end portion of the catheter is located can be determined, since the bending direction and bending radian of the distal end portion of the catheter are not clear, it is actually still difficult to effectively control the action of the catheter.

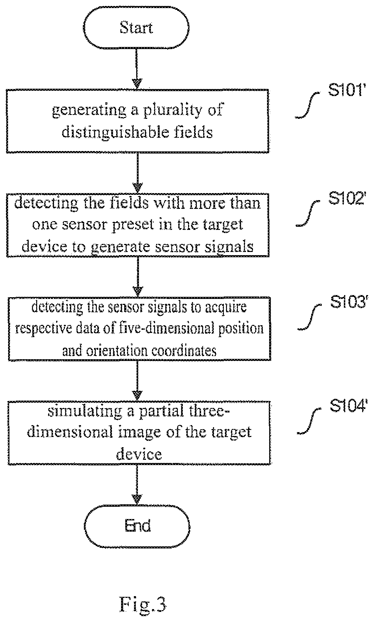

In order to make the intrusive target device provided in the present invention to be more easily controlled and moved, a target device comprising more than one sensor may be further employed. More than one field sensor is preset on different parts of the target device for forming a partial three-dimensional image of the target device. Refer to FIG. 3 for the detailed steps. FIG. 3 schematically shows a flow of a non-limited preferred implementation of the present invention.

At step S101', the positioning signal processing device transmits a cyclic control excitation signal to the magnetic field generating device, so that the magnetic field generating device driven by this drive signal transmits distinguishable AC magnetic fields to the surrounding space including an area above the operation table where the heart of the patient exists.

At step S102', more than one sensor that has been preset in the distal end portion of the catheter and positioned in the heart cavity of the patient and the AC magnetic fields generate electromagnetic induction so as to generate a voltage/current signal. Generally, the more than one sensor preset in the catheter is separated from one another by an equal or unequal distance. Preferably, two sensors that are mutually spaced apart by a certain distance are employed because it is beneficial for reducing cost by controlling the number of sensors.

At step S103' the voltage/current signal is transferred to the positioning signal processing device through the connection between one of the magnetic field sensors and the positioning signal processing device. The positioning signal processing device can conduct synchronous correlation on the control excitation signal and the voltage/current signal induced by the more than one sensor so as to calculate the five-dimensional coordinates of the points in the distal end portion of the catheter where the more than one sensor is located, including the three-dimensional spatial coordinates and two orientation coordinates.

At step S104', a partial three-dimensional image model of the target device is obtained by processing the data of three-dimensional position coordinates and/or data of two orientation coordinates in the obtained data of five-dimensional position and orientation coordinates of the points where the more than one sensor is located in the target device. Since the specification of the target device, and parameters such as the diameter and material of the cardiac catheter, the number of sensors preset in the catheter, the distance between the respective sensors, and the distance between the catheter distal end and the sensors etc are known, and the specifications and positions of working devices or functional devices such as an electrode preset on the catheter etc are also known, it is possible to simulate a partial three-dimensional image model of the target device by using a mathematical simulation method based on these known data in the case where a plurality of points, i.e., positions of a plurality of sensors, on the target device are known. In addition, it is also possible to further display the functional devices such as the electrode preset on the catheter etc on the three-dimensional image model based on the known specifications of various working devices or functional devices.

The obtained partial three-dimensional image model of the target device can be used for the three-dimensional image navigation mode, which can synchronously display changes in the form of the partial three-dimensional image model during the navigation. For example, when the manipulator controls the bending degree of the distal end portion of the cardiac catheter or moves it through a handle, the partial three-dimensional image model of the distal end portion of the catheter will also show synchronous change in the bending shape. Different from the previously described solution of using only one point or symbol to represent the distal end portion of the catheter, this solution can enable the manipulator to clearly know the form of the distal end portion of the target device, such as the bending radian and bending direction of the cardiac catheter, so as to help the manipulator to more effectively position and move the target device. On the other hand, this solution also overcomes the problem of inconvenience of the manipulation that may be caused by the lack of self-rotation angle j data from the other aspects. As described above, in order to simplify the process of acquiring and processing the coordinate data, the present invention employs the solution of regarding the target device as a cylinder, and neglects influences on the manipulation caused by the rotation in an axial direction of the target device by utilizing the characteristic that a cylinder is symmetrical with respect to the axis, so that it is unnecessary to obtain its self-rotation angle j. Although this solution of omitting the self-rotation angle can simplify the six-dimensional data to the five-dimensional data, in the basic implementation that the target device merely comprises one field sensor, it will increase the difficulty in positioning and moving the target device in a certain degree. Since it is impossible to keep the axis of the target device straight in the practical usage, for instance, the distal end portion of the cardiac catheter will be inevitably bent during the manipulation, if there is no indication of the self-rotation angle coordinates j of the catheter along the axis, it will be unable to determine or mark the bending direction of the cardiac catheter. In fact, the conventional measure in the prior art is to indicate the rotation in the axial direction of the catheter by using the change in the self-rotation angle j in the six-dimensional data so as to mark the real bending direction of the catheter. However, in the preferred implementation of the present invention, the relevant problems can be easily overcome because the bending radian and direction of the target device have been vividly simulated as an image, which is obviously more visual and vivid than manually marking the rotation direction of the target device, and more helpful for the manipulator to effectively position and move the target device.

In the particular application, a plurality of distinguishable fields in this implementation respectively have different frequencies or different phases, or have both different frequencies and different phases. Generally, the frequencies of the plurality of distinguishable fields are integer multiples of a given frequency.

The measure for acquiring the data of five-dimensional position and orientation coordinates of the point where the sensor is located on the target device mainly comprises cross-correlating the drive signal to the sensor signal. When the frequencies of the plurality of distinguishable fields are integer multiples of a given frequency, the drive signal and the sensor signal can be cross-correlated, wherein the duration of the cross-correlation is a result obtained by dividing the least common multiple of the integer multiples by the given frequency. The result of the cross-correlation is used for calculating respective contributions of the plurality of distinguishable fields to the signals generated by the sensors, so as to finally obtain the data of five-dimensional position and orientation coordinates.

The person skilled in the art should understand that the technical solution described in this implementation is merely used for exemplification and not for limiting the present invention. The person skilled in the art can replace the technical features mentioned in the technical solution according to the requirement, for example, the magnetic field may either be a continuous AC magnetic field, or a DC pulse magnetic field.

Based on the above technical solution, the method of this implementation may further comprise a step of recording an electrical signal of a current position by using the sensor preset in the target device. For example, the electrical signal of the heart cavity inner wall of the patient can be simultaneously detected with the electrode during the three-dimensional mapping, and then the collected plurality of electrical signals of the heart cavity inner wall are processed in combination with the three-dimensional position data in the corresponding collected five-dimensional data to form a map, which represents the propagation situation of the electrical signals via the tissue of the heart cavity inner wall and is available for view by the doctor.

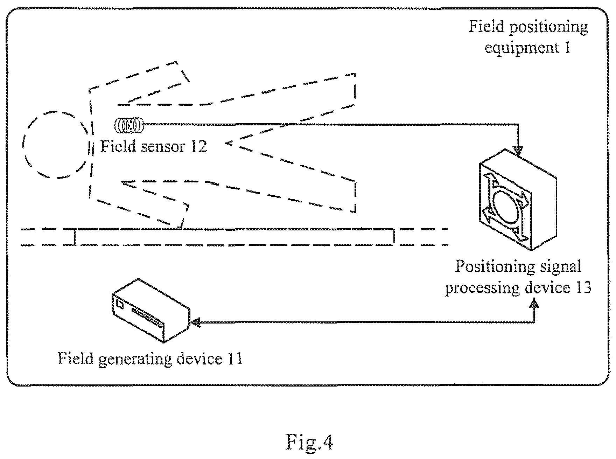

The field positioning equipment according to the second aspect of the present invention will be described below with reference to the schematic system diagram of the field positioning equipment shown in FIG. 4 in combination with another detailed embodiment.

As shown in FIG. 4, the field positioning equipment 1 of the present invention is mainly used for mapping the cavity in a human body, such as a heart cavity, and can create a three-dimensional model of the cavity inner wall of the human body and implement the navigation of a target device in the cavity of the human body. The field positioning equipment 1 comprises: at least one field generating device 11; at least one field sensor 12; and a positioning signal processing device 13.

In the field positioning equipment 1 provided in this embodiment, the field generating device 11 is an AC magnetic field generating device for transmitting an AC magnetic field to a space; the field sensor 12 is a five-dimensional magnetic field sensor comprised of a coil for inducing the AC magnetic field to generate a voltage/current signal. The field generating device 11 and the field sensor 12 are respectively connected to the positioning signal processing device 13. When the positioning signal processing device 13 transmits a cyclic control excitation signal, i.e., a drive signal, to the magnetic field generating device 11, the magnetic field generating device 11 may transmit the AC magnetic field to a space. Generally, the magnetic field generating device 11 is placed below a support table, such as an operation table, so that the transmitted magnetic field can effectively cover the space above the table, and the human cavity of a patient that needs detection can be covered by the magnetic field transmitted by the magnetic field generating device 11 when the patient lies down on the support table. In order to achieve the detection purpose, some certain intrusive measures are needed to allow the field sensor 12 to enter into the human cavity. For example, a catheter is used to enter into the heart cavity through a blood vessel of a human body, with the field sensor 12 being preset in the distal end portion of the catheter. The target device, i.e., the catheter, is not shown in FIG. 4. Generally but not limited, the field sensor 12 may be coaxially placed in the distal end portion of the catheter so that the axis of the sensor 12 and the axis of the catheter coincide.

Taking the three-dimensional detection inside a heart cavity as an example, it is assumed that a patient has lied down on the operation table, and a target device, i.e., the distal end portion of the catheter in which the field sensor 12 is preset has entered into the heart cavity of the patient.

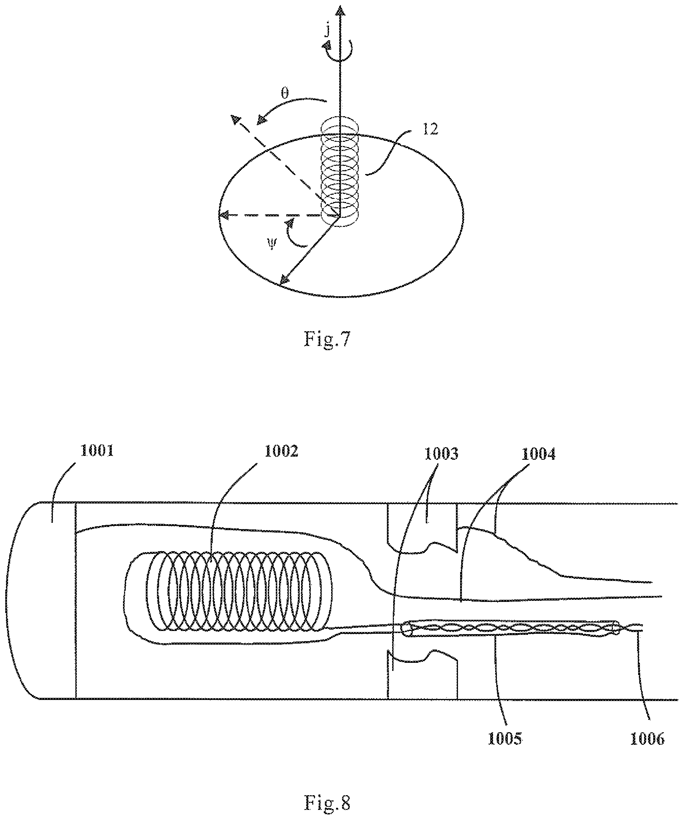

The positioning signal processing device 13 may transmit a cyclic control excitation signal to the magnetic field generating device 11, so that the magnetic field generating device 11 driven by the drive signal transmits distinguishable AC magnetic fields to the surrounding space including an area above the operation table where the heart of the patient exists. The sensor 12 that has been preset in the distal end portion of the catheter and positioned in the heart cavity of the patient may generate electromagnetic induction with the AC magnetic field so as to generate a voltage/current signal. The voltage/current signal is transferred to the positioning signal processing device 13 through the connection between the magnetic field sensor 12 and the positioning signal processing device 13. The positioning signal processing device 13 can synchronously correlate the control excitation signal to the voltage/current signal induced by the sensor 12 so as to calculate five-dimensional coordinates including three-dimensional spatial coordinates and two orientation coordinates of a point where the sensor 12 is located. More specifically, the three-dimensional spatial coordinates mentioned here are the three-dimensional coordinates based on a predetermined three-dimensional coordinate system, and the two orientation coordinates refer to angle coordinates. As shown in FIG. 7, in the present invention, the orientation coordinate showing axial rotation around the center of the sensor coil 12 is defined as self-rotation angle j and the other two angles respectively represent a nutation angle $\theta$ and a procession angle $\psi$ of vertical and horizontal rotation of the sensor coil. In order to simplify the process of acquiring and processing the coordinate data, the distal end portion of the catheter where the sensor 12 of this embodiment is located is designed to be a cylinder or in other shape that is symmetrical along the axis. Accordingly, during acquiring and processing the coordinate data, the distal end portion of the catheter can be regarded as a cylinder. In the precondition of such setting, it may be regarded that the axial rotation of the catheter distal end has no influence on the signal collection, the contact between the catheter distal end and the heart cavity inner wall, and the transmission of treatment signals, and also has no influence on the creation of the three-dimensional image of the heart cavity inner wall, thus it is unnecessary to consider the influence caused by the change in the self-rotation angle j, and only the nutation angle $\theta$ and the procession angle $\psi$ need to be acquired. If not specifically specified, the data of five-dimensional position and orientation coordinates or the five-dimensional coordinates refer to the three-dimensional position coordinates and the nutation angle $\theta$ and the procession angle $\psi$.

With the aforesaid device, it is possible to obtain the data of five-dimensional position and orientation coordinates of the target device where the sensor 12 is located in the cavity of the patient, wherein the position data is used for marking the position of the target device (actually it is the place in the target device where the sensor coil is located, and more specifically, it may be the center of the coil) in the three-dimensional space, and the orientation data is used for marking the orientation of the target device (actually it is the sensor coil in this target device) in the three-dimensional space.

As described above, in order to create a three-dimensional model for the heart cavity inner wall and use the three-dimensional model to navigate the target device in the heart cavity, the target device containing the sensor 12 needs to be moved along the heart cavity inner wall. In this embodiment, the target device is a catheter. The distal end portion of the catheter should touch the heart cavity inner wall during its moving. In order to determine whether the distal end portion of the cardiac catheter touches the heart cavity inner wall, a monitoring electrode and/or a pressure sensor for assisting judgment may be selectively added to the catheter distal end so that the manipulator can make the judgment based on the corresponding feedback parameters: for example, monitoring an electrical signal by using the electrode preset in the distal end portion of the catheter (monitoring the ECG in this implementation), if the distal end portion of the catheter touches the heat inner wall, the magnitude of differential signals monitored by the electrode is relatively big; or, the manipulator may also judge whether the distal end portion of the catheter touches the heart cavity inner wall based on the pressure signal fed back from the pressure sensor at the catheter tip.

By moving the target device containing the field sensor 12 on the heat inner wall for several times, it is able to record and obtain a plurality of sets of corresponding five-dimensional coordinates, which may be used for creating the three-dimensional image model of the heart cavity inner wall. For example, after recording position coordinates of a plurality of sets of specific positions of the human cavity inner wall where the target device stays, it is possible to obtain the three-dimensional image model of the human cavity inner wall by mathematical simulation according to a corresponding anatomical structure. The simulation process of the three-dimensional image model is implemented by a three-dimensional image simulating device, which should be construed as being integrated in the positioning signal processing device 13 in this implementation, so the three-dimensional image simulating device is not shown separately in the figure. The function of this simulating device is to process the aforesaid data of five-dimensional position and orientation coordinates, and creates the three-dimensional image model based on the data of three-dimensional position coordinates and/or data of two orientation coordinates in the data of five-dimensional position and orientation coordinates. Generally, the model of the cavity inner wall can be simulated by merely using the three-dimensional spatial coordinates in the data of five-dimensional coordinates, but in the practical application, since the field sensor 12 is preset in the catheter, there is in fact always a certain distance between the place where the catheter is mounted and the place where the catheter touches the inner wall. In order to simulate the human cavity inner wall more accurately, before acquiring the data of position coordinates for simulating the cavity inner wall , the positioning signal processing device 13 needs to be calibrated for the offset amount to remove the error caused by the distance between the place where the sensor is located and the distal end of the cardiac catheter. In this case, all the data of five-dimensional coordinates, i.e., the three-dimensional coordinate and the two orientation coordinates, need to be used for the calculation of the offset amount to be calibrated. More specifically, in the case where the three-dimensional coordinates of the place where the sensor is located in the cardiac catheter has been obtained, since the distance between the sensor and the distal end of the cardiac catheter is known, it is possible to obtain the three-dimensional coordinates of the point where the catheter actually touches the wall through mathematical calculation only based on the current data of orientation coordinates of the cardiac catheter, i.e., the two orientation coordinates, said three-dimensional coordinates can be used to accurately simulate the cavity inner wall .

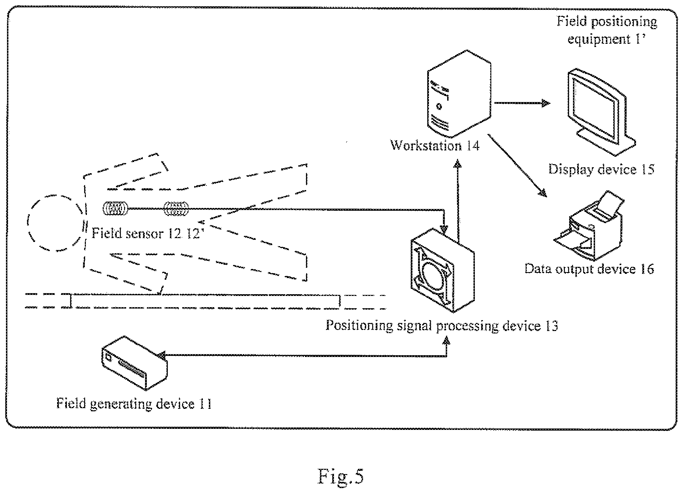

After forming the three-dimensional image of the human cavity inner wall through simulation, the three-dimensional image can be used for vividly positioning and navigating the target device containing the field sensor 12. In this implementation, the target device is the distal end portion of the heart cavity. The positioning signal processing device 13 in this embodiment may be attached with a display device, which is preferably, independent from the positioning signal processing device 13 and cooperates with a workstation for image processing to implement relevant image processing and display functions. Refer to FIG. 5 for details. FIG. 5 shows a field positioning equipment 1' according to one preferred implementation of the present invention. Based on the original field positioning equipment, this field positioning equipment 1' further comprises a workstation 14, a display device 15 and a data output device 16. In this preferred implementation, the positioning signal processing device 13 is used for synchronously correlating the control excitation signal to the voltage/current signal induced by the sensor 12 so as to calculate the five-dimensional coordinates, i.e., the three-dimensional spatial coordinates and two orientation coordinates, of the vortex of the catheter tip, and then transmitting the coordinate data to the workstation 14. The workstation 14 can obtain the three-dimensional image of the heart cavity inner wall by three-dimensional image processing on a plurality of coordinates of the vortex of the catheter distal end obtained by moving the catheter at different positions in the heart cavity for a plurality of times. The three-dimensional image created by the workstation 14 is displayed in the display device 15 for view by the doctor. The doctor may also print the desired image and data through a data output device 16 or export them to a desired medium format for subsequent analysis and processing.

Both the three-dimensional image of the human cavity inner wall and the distal end portion of the catheter determined according to the field sensor 12 can be vividly displayed in the display device 15, and the change of the relative positions of the distal end portion of the catheter in the human cavity can be synchronously reflected. The manipulator can easily determine the position of the catheter tip based on the displayed image, and control the movement and positioning of the catheter through an external control device, such as a control handle at the remote end on the rear part of the catheter.

In the basic solution of this implementation, the target device that is intrusively imbedded into the human cavity can only comprise one sensor 12, or namely one coil. In this case, there is only one position point, i.e., the position where the coil of the sensor 12 is located that can be used for determining the position of the target device. Accordingly, in the above three-dimensional image navigation mode, the point or symbol represented by only one such sensor 12 being used for displaying the position of the target device is visually displayed, and it cannot reflect the detailed form of the target device. Taking the cardiac catheter mentioned in this implementation as an example, this catheter is in an elongated cylindrical shape, and a segment thereof close to the distal end portion can be controlled by a remote handle, and bent or straightened in a certain radian range. After the catheter has entered into the heart cavity, the distal end portion of the catheter is generally kept in a bending shape of a certain radian so that its distal end can better abut against or hook up the heat inner wall. If only one sensor is used, it will be unable to display the bending form of the catheter. In the practical application, however, the doctor may not effectively manipulate the target device because he/she does not know the detailed form of the target device. For example, in the case where the cardiac catheter is inside the heart cavity, even if it is able to determine the position where the distal end portion of the catheter is located, since the bending direction and the bending radian of the distal end portion of the catheter are not clear, it is actually still difficult to effectively control the action of the catheter.

A field positioning equipment according to one preferred implementation of the present invention will be described below with reference to FIG. 5. In order to make the intrusive target device provided in the present invention to be more easily controlled and moved, this implementation further provides a target device comprising more than one sensor, i.e., field sensors 12 and 12'. The field sensors 12 and 12' are preset at different positions of the cardiac catheter for forming a partial three-dimensional image of the target device. The mounting mode of the field sensor 12 is the same as the embodiment shown in FIG. 4, and the position of the sensor 12 with respect to the catheter distal end is fixed, and other parts of the distal end portion except for the part where the sensors are mounted are deformable. The cardiac catheter is not displayed in FIG. 5 either. Actually, the number of the field sensors is not limited to two, and it may be more than two according to the actual requirement. Theoretically, the more sensors mounted on the target device, the more accurate the finally simulated partial three-dimensional image of the target device is. However, in order to control cost, the present invention preferably employs two sensors that are mutually spaced apart by a certain distance along the catheter. In the case where the specification parameters of the catheter are known, it has been able to relatively accurately simulate the partial three-dimensional image of the catheter by merely using two sensors. In this technical solution, the field sensors 12 and 12' that are preset near the catheter distal end and are spaced apart by a certain distance along the catheter can respectively generate electromagnetic induction with the AC magnetic fields generated by the field generating device 11, thereby respectively generating voltage/current signals. If more than two sensors are used, these sensors may be mutually spaced apart by an equal or unequal distance along the catheter.

The voltage/current signals are transferred to the positioning signal processing device 13 through the connections between the field sensors 12 and 12' and the positioning signal processing device 13. The positioning signal processing device 13 can synchronously correlate the control excitation signal to the voltage/current signals induced by the sensors 12 and 12' so as to respectively calculate five-dimensional coordinates including three-dimensional spatial coordinates and two orientation coordinates of the points where the sensors 12 and 12' are located. In the preferred implementation shown in FIG. 5, the workstation 14 may be used for implementing the function of the previously described three-dimensional image simulating device. In other words, the workstation 14 includes the three-dimensional image simulating device for processing the data of five-dimensional position and orientation coordinates, and creates a three-dimensional image model based on the data of three-dimensional position coordinates and/or data of two orientation coordinates in the data of five-dimensional position and orientation coordinates.

Preferably, the obtained data of five-dimensional position and orientation coordinates of the points where the sensors 12 and 12' are located in the target device are transmitted to the workstation 14. The three-dimensional image simulating device in the workstation 14 can create in real time the partial three-dimensional image model of the catheter in the heart cavity according to the obtained coordinate data. Since the specification of the target device, such as a diameter and material of the cardiac catheter, the number of the sensors preset in the catheter, the distance between the respective sensors, and the distance between the catheter distal end and the sensors, are known, and the specifications and positions of the functional devices, such as an electrode preset on the catheter are also known, it is possible to partially simulate the three-dimensional image model of the target device near the positions of these sensors by using a mathematical simulation method based on these known data in the case where the positions of a plurality of sensors on the target device are known. Besides, it is also possible to further display on the three-dimensional image model the functional devices such as the electrode preset on the catheter based on the known specifications of other working devices or functional devices in the catheter.

The obtained partial three-dimensional image model of the target device containing the field sensors 12 and 12' can be used for the three-dimensional image navigation, which can synchronously display changes in the form of the partial three-dimensional image model of this target device during the navigation. For example, when the manipulator controls the bending degree of the distal end portion of the cardiac catheter or moves it through a handle, the partial three-dimensional image model of the distal end portion of the catheter will also show synchronous changes in a bending shape. Different from the previously described solution of using only one point or symbol to represent the distal end portion of the catheter, this solution can enable the manipulator to clearly know the form of the distal end portion of the target device, such as the bending radian and the bending direction of the cardiac catheter, so as to help the manipulator to more effectively position and move the target device.

On the other hand, this solution also overcomes the problem of inconvenience of manipulation that may be caused by the lack of self-rotation angle j data from other aspects. As described above, in order to simplify the process of acquiring and processing the coordinate data, the present invention employs the solution of regarding the target device as a cylinder, and neglects influences on the manipulation caused by the rotation in an axial direction of the target device by utilizing the characteristic that a cylinder is symmetrical with respect to the axis, so that it is unnecessary to obtain its self-rotation angle j. Although this solution of omitting the self-rotation angle can simplify the six-dimensional data to the five-dimensional data, in the basic implementation in which the target device merely comprises one field sensor, it will increase the difficulty in positioning and moving the target device in a certain degree. Since it is impossible to keep the axis of the target device straight in the practical usage, for instance, the distal end portion of the cardiac catheter will be inevitably bent during the manipulation, if there is no indication of the self-rotation angle coordinate j of the catheter along the axis, it is unable to determine or mark the bending direction of the cardiac catheter. In fact, the conventional measure in the prior art is to indicate the axial rotation of the catheter by using the change in the self-rotation angle j in the six-dimensional data so as to mark the actual bending direction of the catheter. However, in the preferred implementation of the present invention, the relevant problems can be easily overcome because the bending radian and direction of the target device have been vividly simulated as an image, which is obviously more visual and vivid than manually marking the rotation direction of the target device, and more helpful for the manipulator to effectively position and move the target device.

As for typically displaying a bending shape, two field sensors need to be mounted in the target device, such as a cardiac catheter, wherein one is close to the distal end of the target device, for instance, about 3 to 10mm away from the distal end of the cardiac catheter, and the other is farther from the distal end of the target device, for instance, about 80 to 120 mm from the distal end of the cardiac catheter. Certainly, three or more field sensors may be mounted in the catheter, so that the display of the bending shape will be more vivid. However, the cost of corresponding devices will increase, and the mounting will become complicated.

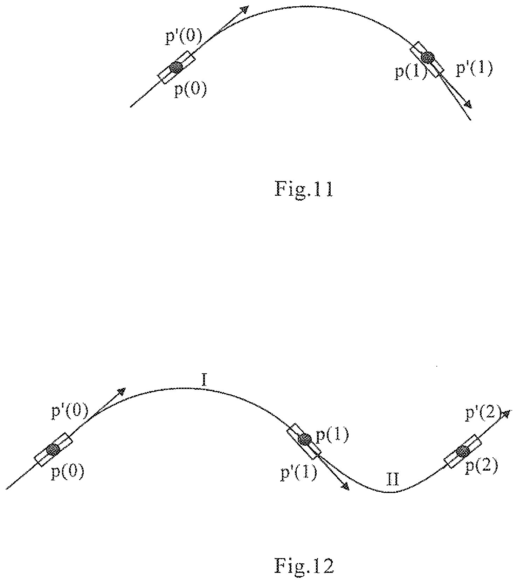

In order to describe the technical solution of the present invention in more detail, the principle and progress of forming the partial three-dimensional image model of the target device will be further described below with reference to FIGS. 11 and 12.

In the case where two field sensors are preset in the target device, as described above, since the distance between the field sensors is a fixed value, the bending track of the catheter can be calculated and simulated by using the following algorithm based on the known parameters of the two field sensors:

1. The cubic splines of curved segments of two sensors approximate.

Referring to FIG. 11, the positions of the field sensors indicated by two rectangular blocks and the bending shape of the catheter are shown in FIG. 11. Assuming that the bending track of the catheter is a simple cubic curve, i.e., in the shape shown by the curve in FIG. 11, the curved segments mounted with the field sensors are regularized, wherein the arc length t of the curve is used as a parameter, and the value range of t is set as [0, 1]. Without losing generality, the equation for the curved segment is set as:

$$p(t) = at^3 + bt^2 + ct + d \qquad \text{(Formula 2-1)}$$

There are four unknown parameters in the equation, and four conditions are needed to find the solution. Directions of p(0), p(1) as well as p'(0) and p'(1) can be acquired in the positioning system, but values of p'(0) and p'(1) cannot be known. The values of p'(0) and p'(1) depend on the material, length of the catheter and the distance between the two field sensors. In this implementation, solutions for p'(0) and p'(1) are obtained by using the minimum energy curve method so as to obtain a relatively satisfying result of the bending model. Refer to the third part below for details.

Now assuming that p(0), p(l) as well as p'(0) and p'(1) are all known, and the equation satisfies four conditions when t = 0, 1, coefficients a, b, c, d in the formula are distributed as follows:

$$\begin{cases} a = 2t^3 - 3t^2 + 1 \\ b = t^3 - 2t^2 + t \\ c = -2t^3 + 3t^2 \\ d = t^3 - t^2 \end{cases} \qquad \text{(Formula 2-2)}$$

such that Formula 2-1 can be written as:

$$p(t) = (2t^3 - 3t^2 + 1)p(0) + (t^3 - 2t^2 + t)p'(0) + (-2t^3 + 3t^2)p(1) + (t^3 - t^2)p'(1) \qquad \text{(Formula 2-3)}$$

Formula 2-3 can also be written in a matrix:

$$p(t) = \begin{bmatrix} t^3 & t^2 & t & 1 \end{bmatrix} \begin{bmatrix} 2 & -2 & 1 & 1 \\ -3 & 3 & -2 & -1 \\ 0 & 0 & 1 & 0 \\ 1 & 0 & 0 & 0 \end{bmatrix} \begin{bmatrix} p(0) \\ p(1) \\ p'(0) \\ p'(1) \end{bmatrix} \qquad \text{(Formula 2-4)}$$

Therefore, it is able to obtain the bending shape of the catheter, i.e., the partial three-dimensional image of the target device.

2. The cubic splines of curved segments of a plurality of sensors approximate.

When the bending of the catheter is relatively complicated, in order to more accurately and vividly describe the bending information of the catheter, more than two field sensors can be mounted to divide the bending shape of the catheter into a plurality of segments of simple three-dimensional curves, as shown in FIG. 12. In a set of given points (p(k), p'(k)), k = 0, 1, 2, ..., n, n+1 given points are divided into groups each having two adjacent points, such as [p(i-1), p(i)]. After performing regularization on segments of each group, the same method used in the previously described simulation process is used and a cubic Hermite spline curve is used for approximation so as to obtain segmented cubic spline curves connecting all given points. The tangent directions of common points between two segments of curves are consistent, and lengths of the tangents are the same, so that the two segments of curves are guaranteed to be the first-order smoothly continuous.

3. A method of measuring tangent vector modulus length

Since the feedback information of the magnetic induction coil in the field sensor merely includes a spatial position and direction of the coil, the spatial position and bending direction of the mounting point of the catheter mounted with the field sensor can be determined, however it is unable to obtain the modulus length of the tangent vector indicating the bending direction of this point through the positioning system. Factors that influence the modulus length of the tangent vector include material, length, and spatial form of the catheter. In order to find solutions for modulus lengths of curve vectors on both ends of a single segment of curve, two known conditions are needed, Assuming that the curvature and curvature radius of a curve are k(t) and p(t), respectively, internal energy of the curve is calculated by using Formula 2-5:

$$E = \int_0^1 k^2(t)\,ds = \int_\alpha^\beta \frac{\rho(t)d\theta}{\rho^2(t)} = \int_\alpha^\beta \frac{d\theta}{\rho(t)} \qquad \text{(Formula 2-5)}$$

Set the modulus lengths of the tangent vectors on both ends of the curve to make the internal energy to be the minimum so as to obtain a known condition. Besides, the curve length can be calculated by using Formula 2-6:

$$\int_\alpha^\beta \rho(t)d\theta = L \qquad \text{(Formula 2-6)}$$

Since the lengths of segments of the curve mounted with field sensors are constant, a second known condition is obtained, so that the modulus length of the tangent vector on both ends of the curve segment is solved.

In the particular application of other aspects, the plurality of distinguishable fields generated by the field generating device in any implementation of the present invention respectively have different frequencies or different phases, or have both different frequencies and different phases. Generally, the frequencies of the plurality of distinguishable fields are integer multiples of a given frequency.

The measure for acquiring the data of five-dimensional position and orientation coordinates of the point where any sensor, including the field sensors 12 and 12' etc. is located on the target device mainly comprises cross-correlating the drive signal to the sensor signal. When the frequencies of the plurality of distinguishable fields are integer multiples of a given frequency, the drive signal and the sensor signal can be cross-correlated, wherein duration of the cross-correlation is a result obtained by dividing the least common multiple of the integer multiples by the given frequency. The result of the cross-correlation is used for calculating respective contributions of the plurality of distinguishable fields to the signals generated by the sensors, so as to eventually obtain the data of five-dimensional position and orientation coordinates.

The person skilled in the art should understand that the technical solution described in this implementation is merely used for exemplification and not for limiting the present invention. The person skilled in the art can replace the technical features mentioned in the technical solution according to the requirement, for example, the magnetic field may either be a continuous AC magnetic field, or a DC pulse magnetic field.

Based on the above technical solution, the method of this implementation may further comprise a step of presetting an electrode in the target device for recording an electrical signal of a current position. For example, the electrode suitable for detecting the electrical signal of the heart cavity inner wall of a patient is added in the target device, and a plurality of electrical signals of the heart cavity inner wall collected by the electrode can be processed in combination with the three-dimensional position data in the corresponding collected five-dimensional data to form a map, which represents the propagation situation of the electrical signals via the tissue of the heart cavity inner wall.

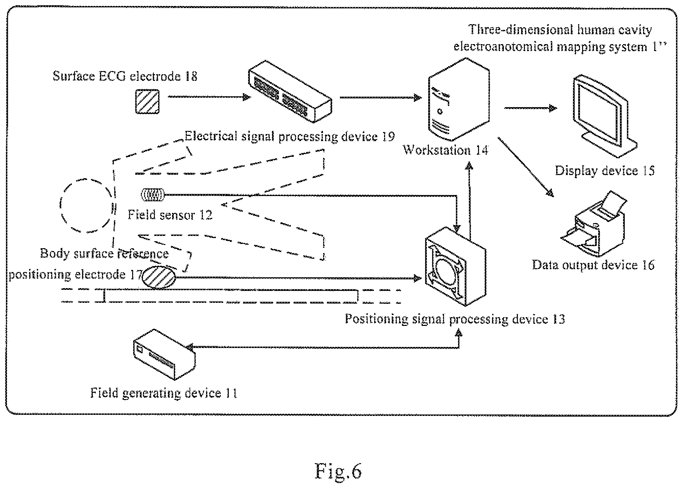

On the basis of the above-described three-dimensional mapping method and the corresponding field positioning equipment, the present invention may be further used for creating a more improved three-dimensional human cavity electroanotomical mapping system. A three-dimensional human cavity electroanotomical mapping system provided according to a third aspect of the present invention will be described below with reference to the three-dimensional human cavity electroanotomical mapping system 1" shown in FIG. 6. This system is mainly different from the original field positioning equipment in the addition of an electrical signal processing device for monitoring various electrical signals of a human body, and of a body surface reference positioning electrode for assisting the field positioning equipment of the present invention.

In the three-dimensional human cavity electroanotomical mapping system 1" of this implementation, the method and equipment used for human cavity three-dimensional mapping are the same as that in the previous implementation. Actually, this three-dimensional human cavity electroanotomical mapping system 1" may further comprise any solution obtained by modifying the method and equipment described in the previous implementation. For example, a three-dimensional human cavity electroanotomical mapping system may be created based on a field positioning equipment comprised of the basic field generating device 11, the field sensor 12 and the positioning signal processing device 13, or may also be created based on a field positioning equipment further containing one or more of the workstation 14, the display device 15, and the data output device 16. As shown in FIG. 6, the three-dimensional human cavity electroanotomical mapping system 1" provided in this implementation further comprises: a body surface reference positioning electrode 17; a Surface ECG (SECG) electrode 18, and an electrical signal processing device 19, wherein the body surface reference positioning electrode 17 is connected to the positioning signal processing device 13, the SECG electrode 18 is connected to the electrical signal processing device 19, and the electrical signal processing device 19 is connected to the workstation 14.

During the practical application, the SECG electrode 18 is connected to a position on the surface of a human body to collect a SECG signal of the human body, which is then input to the electrical signal processing device 19. The target device, i.e., the far end portion of the catheter containing the field sensor 12 is entered into the heart cavity through a blood vessel of the human body, and the near end portion of the catheter is outside the human body. The far end of the catheter is connected to the heart cavity inner wall through a preset catheter electrode to collect IECG signals on the heart cavity inner wall, and the electrical signals of the heart cavity inner wall are transmitted to the electrical signal processing device 19 through a catheter cable. The electrical signal processing device 19 obtains analog SECG and IECG signals, and obtains digital SEG and IECG signals after internal amplification, filtering, digitalizing, and waveform extraction, and then transmits the digital signals to the workstation 14.

The positioning signal processing device 13 transmits a cyclic control excitation signal to the field generating device 11, which is located near the heart of the human body and transmits AC magnetic fields to the space. The heart of the human body is within the available accuracy range of the magnetic fields generated by the field generating device. The field sensor 12 inside the catheter distal end in the heart of the patient induces the AC magnetic fields to generate a voltage/current signal, which is then transmitted to the positioning signal processing device 13. The positioning signal processing device 13 synchronously correlates the control excitation signal to the voltage/current signal induced by the sensor to calculate five-dimensional coordinates, i.e., three spatial position coordinates and two orientation coordinates, of the vertex of the catheter tip, and then transmits the coordinate data to the workstation 14.

The workstation 14 is used for processing the obtained ECG signals and the spatial position signals of the catheter, extracting the SECG R wave at the end of a Cardiac Diastole period as a synchronous gating signal, and synchronously collecting the spatial five-dimensional coordinates of the vertex of the catheter tip. By moving the catheter in different positions for a plurality of times in the heart cavity, a plurality of vertex coordinates of the catheter can be acquired. A three-dimensional anatomical image of the heart cavity can be acquired by three-dimensional imaging process of the workstation 14. A corresponding three-dimensional electroanatomical image of the heart cavity may be acquired by incorporating the IECG signal of each point synchronously collected by the electrode at the catheter distal end into the three-dimensional heart cavity image. Meanwhile, the workstation 14 may also create in real-time a three-dimensional image of the catheter in the heart cavity based on the obtained coordinate data, thereby navigating the catheter in real-time and visually guiding the doctor to manipulate the catheter to move the distal end portion of the catheter toward the desired position.

The three-dimensional image created by the workstation 14 is displayed on the display device 15 for view by the doctor. The doctor may also print the desired image and data through the data output device 16 or export them to a desired medium format for subsequent analysis and processing.

More specifically, the distal end portion of the catheter mounted with the field sensor described in any one of the equipments or systems in the present invention may be mounted with a plurality of metal electrodes, typically platinum electrodes, for collecting ECG signals on the heart cavity inner wall. The lengths of these electrodes are within a range of 1 to 8 mm, and typically the length of the electrode closest to the distal end is 4 mm, and others are 1.5 mm. The distal end electrode synchronously transmits energy signals, such as a radiofrequency ablation current and electric excitation signal, to the heart cavity inner wall. There is a tubular device in the catheter handle and the catheter cavity and there are a plurality of micropores on the electrodes of the catheter distal end for transferring a liquid, for example, physiological saline for reducing temperature when radiofrequency ablating the target point, or a therapeutic solution, to the surface of the heart cavity inner wall.

The distal end portion of the catheter mounted with the field sensors described in any one of the equipments or systems in the present invention may be mounted with a temperature sensor, which is typically a thermocouple or a thermal sensitive temperature sensor, for measuring the temperature of the point of the inner wall tissue of the heart cavity where the catheter distal end touches, and providing a feedback signal to a radiofrequency ablation controller to control emission of the radiofrequency ablation energy.

The distal end portion of the catheter mounted with the field sensors described in any one of the equipments or systems in the present invention may be mounted with a pressure sensor for measuring how good a contact between the catheter distal end and the myocardial tissue is.

In order to make it easier to understand the detailed structure of the catheter employed in the various equipments or systems in the present invention, the structure of the catheter will be described below with reference to the drawings. Since the distal end portion of the catheter constitutes the so-called target device in general meaning of the present invention, the provided drawings are partial schematic diagrams of the distal end portion of the catheter.

FIG. 8 is a sectional view of the distal end portion of the cardiac catheter according to one implementation of the present invention. The cardiac catheter comprises at its tip: a catheter distal end electrode 1001, a field sensor 1002, and a catheter annular electrode 1003. As shown in FIG. 8, the catheter distal end electrode 1001 and the catheter annular electrode 1003 are respectively connected to the rear part of the cardiac catheter through an electrode wire 1004, and they can be used as the electrodes in any one of the previously described equipments or systems. The field sensor 1002 contains a coil, and is connected to the rear part of the cardiac catheter through a sensor electrical wire 1006, which is wrapped by a metallic shielding layer 1005.

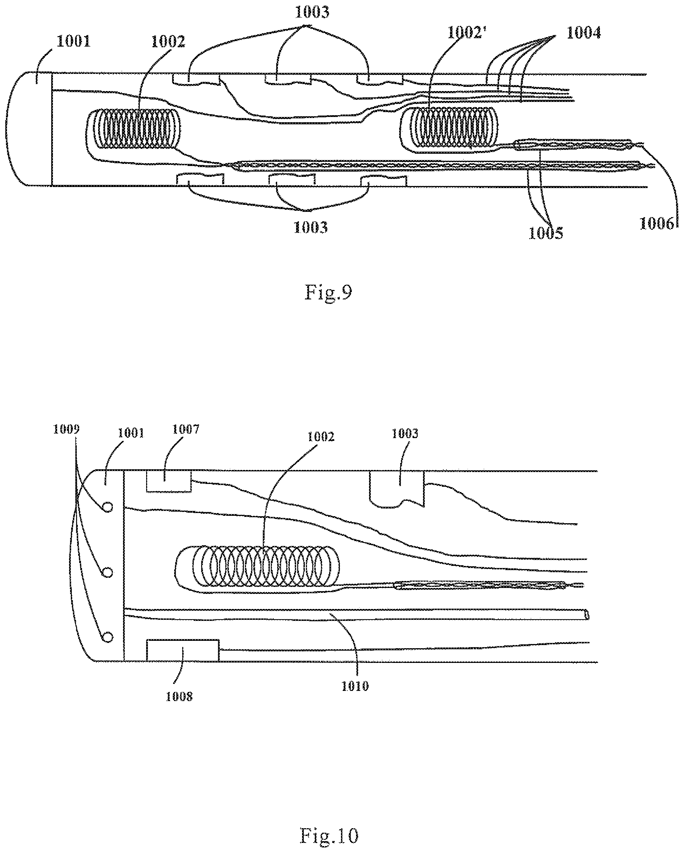

FIG. 9 is a sectional view of the distal end portion of the cardiac catheter according to one preferred implementation of the present invention. The cardiac catheter comprises at its tip: a catheter distal end electrode 1001, field sensors 1002 and 1002', and a catheter annular electrode 1003. As shown in FIG. 9, the cardiac catheter contains two field sensors therein that may be used for implementing any aforesaid equipment or system that can simulate the partial three-dimensional image of the distal end portion of the catheter. The other components have the same or similar functions as those contained in the catheter shown in FIG. 8.

FIG. 10 is a sectional view of the distal end portion of the cardiac catheter according to another preferred implementation of the present invention. The cardiac catheter is mainly used in any one of the three-dimensional human cavity electroanatomical mapping systems provided in the present invention, and in comparison with the design schemes of the previous two types of catheters, this design scheme may implement more functions. The cardiac catheter comprises at its distal end portion: a temperature sensor 1007, which may be used as the temperature sensor of any one of the aforesaid equipments or systems; a pressure sensor 1008, which may be used as the pressure sensor of any one of the aforesaid equipments or systems; and a liquid exiting micropore 1009 for outputting a liquid to the outside through a tube 1010 connected thereto. The other components shown in FIG. 10 have the same or similar functions as the corresponding parts shown in FIG. 8.

Any one of the equipments or systems mentioned in the present invention further includes a body surface reference positioning electrode, which is a non-intrusive instrument and mounted on the body surface of a patient, typically, on the back of the patient, and cannot be easily moved. The distal end portion of the reference electrode is mounted with at least two micro electromagnetic field sensors for inducing varying electromagnetic fields emitted by the electromagnetic field generating device to generate a voltage/current signal, which represents a signal of three-dimensional spatial coordinates and three orientation coordinates for one point on the distal end portion of the reference positioning electrode. The body surface reference positioning electrode provides a reference point in the space that is still with respect to the patient (the reference electrode can be moved with the patient) in the magnetic positioning system. The present three-dimensional mapping system takes the coordinates of the reference point as a reference value and performs difference processing on the spatial coordinates of the catheter inside the patient's heart that are obtained in real time and the spatial coordinates of the reference point so as to obtain the data of three-dimensional position coordinates . This relative coordinate data is used as spatial position data for creating the three-dimensional image of the heart cavity inner wall. In other words, taking one point on the reference electrode as the origin of a coordinate system, the relative position between the origin and the heart is constant, and the spatial coordinates of each of respective points on the catheter form, by calculating with respect to the coordinates of the origin, the coordinate values in the coordinate system represented by this origin, wherein said coordinate values are used for creating a three-dimensional anatomical graph of the heart and three-dimensional imaging and navigation for the catheter.

The equipments or system in the present invention may further comprise an intracardiac reference catheter, which is an existing ordinary electrophysiology diagnosis catheter already applied in the market and generally does not contain the above-described sensors and auxiliary sensors such as the pressure or temperature sensor etc. The distal end portion of the catheter merely includes a plurality of ECG metallic electrodes for detecting ECG signals on the heart cavity inner wall. The catheter distal end is located at a fixed position on the heart cavity inner wall, typically near the coronary sinus of the heart, for generating a reference time signal for the heart beating ECG. From the ECG signals collected by the electrodes of the catheter and the ECG signals connected by the intracardiac reference catheter, according to the time sequence of occurrence of the same characteristic waveforms for the ECG, i.e., the time sequence when the ECG signals pass through two points where the two catheter electrodes make collection in the heart cavity inner wall, a difference between the two time is calculated. Vividly speaking, the normal cardiac pacing electrical signal is originated from the sinoatrial node, and this signal is propagated from the atrial to respective directions of the ventricular via a conduction path. Two monitoring points are placed on its conduction path to monitor the time when the ECG signal respectively arrives at the two monitoring points. A map of propagation of the electrical signal through the inner wall tissue of the heart cavity is formed by comparing a plurality of time difference parameters, in combination with the collected data of three-dimensional position coordinates and/or data of two orientation coordinates in the aforesaid data of five-dimensional position and orientation coordinates.

The equipments or system in the present invention may further comprise an electrical signal processing device, which may acquire an electrophysiological signal of an object and provide the processed signal to the outside. The electrical signal processing device comprises:

a SECG electrode and an adaptive cable for collecting multiple leads of SECG signals, typically, 12-lead SECG signals;
an intracardiac catheter electrode and an adaptive cable for collecting multiple channels of IECG signals, typically, 16 to 32-channel IECG signals;
a collecting circuit of one or more channels of temperature signals for collecting temperature signals;
a collecting circuit of one or more channels of pressure signals for collecting pressure signals;
a pre-amplifying module connected to the various signal collecting cables to amplify and filter the collected signals;
a collecting module connected to the pre-amplifying module for digitally collecting the analog signals output from the pre-amplifying module; and
a digital filtering module to digitally filter the digitalized signals and extract waveforms to obtain the aforesaid various signals.

A communication output module uploads the extracted digitalized signals to the workstation through communication cables. The communication output interface can be USB, Ethernet, optical fiber, serial port, parallel port, Bluetooth and so on.

The electrical signal processing device further comprises a dedicated isolation power module for ensuring that its electric safety index complies with the requirement of National Standard.

The electrical signal processing device may output one channel of signal for providing a synchronous signal for the creation of a three-dimensional image of the heart cavity. Since the heart beats continuously, and for the convenience of view, the created three-dimensional image of the heart cavity needs to be in a still state, this needs to select the data at a certain fixed moment during a heart beating period, or needs the data in a certain fixed state to make drawing. By acquiring this channel of synchronous signal, the aforesaid data can be obtained. It is known that when CT and MRI equipments scan a heart model of a human body, a feature point of the SECG waveform is generally used as a gating triggering point, such as the highest point of R wave. One preferred embodiment of the present system also employs a certain feature point on R wave, such as the highest point of R wave, to start the creation of the three-dimensional graph of a heart cavity. The positioning data of respective points on the catheter and the reference electrode at corresponding moments obtained at the highest point of R wave is used to create the three-dimensional graph. The synchronous signal in the present invention may also be the signals of invasive blood pressure, non-invasive blood pressure, and blood oxygen saturation, and these signals are more related to the mechanical activity of the heart. The highest points of these signal waveforms or the points with the highest rising speed are used as the gating points. Typically, various gating synchronous signal points are selected as the corresponding points at the end of Cardiac Diastole period, because at this moment, the heart volume is the biggest, the heart cavity is in a relatively short still state, and keeping a short stable state in an entire heart beating period facilitates acquiring the data of these points. For the human cavity whose shape is not periodically changed, the data collecting chance can not be determined by using the above synchronous signal when creating the three-dimensional image model for its inner wall, and the mode of data collection should be determined according to the practical situation. The person skilled in the art should understand that the three-dimensional mapping equipment or system in the present invention may be correspondingly improved according to the actually determined data collection mode.

The electrical signal processing device is used for acquiring various electrophysiological data of a patient. ECG signals on the heart cavity inner wall collected by the electrodes on the catheter are acquired synchronously while acquiring the spatial position signal of each point on the heart cavity inner wall through the catheter. The system combines the spatial position signals and the ECG signals, and thus it is possible to form a three-dimensional electroanatomical graph of the heart cavity in the three-dimensional system. This graph can not only provide a visual three-dimensional heart cavity structure for the doctor, but also display ECG change information of different positions in the heart cavity, such as plenty of electroanatomical information including pressure graph, potential graph, activation sequence graph of the heat cavity and so on, which is very helpful for the doctor to analyze pathogenic mechanism of arrhythmia and accurately locate the focal target so as to totally ablate the focal area.

Since this system has a function of processing various electrophysiological signals and integrates the recreation of a three-dimensional image with the electrophysiological function, it can effectively overcome the following disadvantages: when using the conventional field positioning equipment in clinic, since the conventional field positioning equipment cannot provide the desired plenty of electroanatomical information, a conventional multi-channel physiological recorder usually needs to be equipped, thus resulting in a complicated connection between the systems, and necessary operation data and information unable to be integrally stored and reproduced, which brings inconvenience to the doctor. Meanwhile, two systems inevitably cause complicated manipulation, an increased number of staff, and increased cost, and more devices occupy more increasingly crowded space in the operation room. The system in the present invention can effectively avoid and overcome the existing disadvantages.

The three-dimensional mapping system further comprises a workstation (computer) having a plurality of data interfaces, and it can read data output from the magnetic positioning system and the electroanatomical signal processing device, and meanwhile calculates and processes these data. By continuously acquiring the electrophysiological signals and positioning signals, the workstation can create a three-dimensional electroanatomical structure image of the heart cavity and meanwhile create a three-dimensional navigation image of the catheter for implementing real-time navigation.

The workstation further comprises an input interface for inputting MRI/CT three-dimensional image data of the heart cavity. After creating the three-dimensional model image of the heart cavity, this image is aligned with the created three-dimensional heart cavity structure to correct and assist to determine the structural feature of the heart. The input interface may be the known interfaces such as a CD-ROM drive interface, an USB interface, or an Ethernet interface, or an optical fiber interface, through which the MRI/CT data are imported into the present three-dimensional system.

The workstation further comprises a display device that can display the above image content and the processing progress. The display device can either display simultaneously on the same screen the three-dimensional image processing information and the electrical signal waveform data information, or respectively display the three-dimensional image and electrical signal information on a plurality of screens. The display device may further comprise a screen splitter so that the screen content can be displayed simultaneously on another screen at the remote. This is very necessary when the workstation needs to be installed outside the operation room, for example, in the observation room, because a manipulating doctor performs manipulation beside the workstation, while surgeons are still in the operating room and they also need view the displayed information.

The workstation further comprises a data printing output module for outputting operation data. Typically, the data printing output module is an optical disc recorder, an MO memory, a large-capacity non-volatile memory, a hard disc drive and so on. It is also possible to print the desired data, image and report.

**Claims**

1. A field positioning equipment (1) for determining a position and orientation of a cardiac catheter with respect to a reference system, comprising at least one field generating device (11), which, under the excitation of a drive signal, generates distinguishable fields; at least one field sensor (12, 12', 1002, 1002') located in said cardiac catheter for detecting the distinguishable fields to generate a sensor signal; a positioning signal processing device (13) for transmitting the drive signal to said field generating device (11) and detecting an output of said field sensor signal so as to generate data of five-dimensional position and orientation coordinates of the position on said cardiac catheter where the sensor (12, 12', 1002, 1002') is located, each group of obtained data of position coordinates is calibrated for offset amount before being used to simulate a cavity inner wall so as to remove the error caused by a distance between the place where the sensor is located and the tip of said cardiac catheter.

2. The field positioning equipment according to claim 1, wherein said cardiac catheter of the equipment comprises more than one said field sensor, and said more than one sensor is mutually spaced apart by an equal or unequal distance.

3. The field positioning equipment according to claim 1 or 2, wherein the plurality of distinguishable fields generated by said field generating device respectively have different frequencies or different phases, or have both different frequencies and different phases, wherein said fields are continuous AC magnetic fields or periodically varied DC pulse magnetic fields.

4. The field positioning equipment according to claim 3, wherein said positioning signal processing device cross-correlates said drive signal with said sensor signal.

5. The field positioning equipment according to claim 1 or 2, further comprising a pressure sensor (1008) for measuring how good a contact between the cardiac catheter and said human cavity inner wall is or further comprising a temperature sensor (1007) for measuring a temperature of a place where the temperature sensor (1007) is located so as to control release of electrical energy based on a temperature signal.

6. The field positioning equipment according to claim 1 or 2, wherein said cardiac catheter further comprises an electrode for acquiring an electrical signal of the heart cavity inner wall of the position where said cardiac catheter is located.

7. The field positioning equipment according to claim 6, further comprising: an electrical signal processing device for processing the electrical signal acquired by said electrode such that said electrical signal is combined with data of three-dimensional position coordinates and/or data of two orientation coordinates in the data of five-dimensional position and orientation coordinates of a plurality of points collected by the positioning signal processing device to form a map indicating the transmission situation of the electrical signal via tissue of the heart cavity inner wall.

8. A human cavity inner wall three-dimensional mapping method for determining a position and orientation of a cardiac catheter with respect to a reference system, said method comprising steps of (a) generating a plurality of distinguishable fields by using a drive signal; (b) detecting the distinguishable fields by at least one sensor preset in said cardiac catheter so as to generate a sensor signal; (c) detecting an output of the sensor signal to acquire data of five-dimensional position and orientation coordinates of the position on said cardiac catheter where the sensor is located, the data of five-dimensional position and orientation coordinates contains data of three-dimensional position coordinates and data of two orientation coordinates, each group of obtained data of position coordinates is calibrated for offset amount before being used to simulate a cavity inner wall so as to remove the error caused by a distance between the place where the sensor is located and the tip of said cardiac catheter.

9. The method according to claim 8, wherein said cardiac catheter is preset with more than one said sensor, by means of which, the method further comprises steps of:

   (g) acquiring data of five-dimensional position and orientation coordinates of the position on the cardiac catheter where the more than one sensor is located using the data respectively measured by the more than one sensor preset in said cardiac catheter; and
   (h) acquiring a partial or whole three-dimensional image model of the cardiac catheter by processing the acquired data of five-dimensional position and orientation coordinates of the position on the cardiac catheter where the more than one sensor is located.

**EP 2 409 643 B1**

10. The method according to claim 9, wherein the more than one said sensor preset in said cardiac catheter is mutually spaced apart by an equal or unequal distance.

11. The method according to claim 10, wherein said cardiac catheter is preset with two sensors that are mutually spaced apart by a certain distance.

12. The method according to any one of claims 8 to 11, further comprising the step of: acquiring an electrical signal of the human cavity of the position where said cardiac catheter is located by using an electrode preset in said cardiac catheter.

13. The method according to claim 12, further comprising the step of: forming, according to a time sequence of acquiring said electrical signal, a map indicating the transmission situation of the electrical signal via tissue of the human cavity inner wall by combining the collected plurality of said electrical signals of the human cavity inner wall with the data of the three-dimensional positions in the correspondingly collected five-dimensional data and processing them.

14. The field positioning equipment according to claim 1, further comprising: a body surface reference positioning electrode (17) mounted on a surface part of a human body, for determining data of three-dimensional positions and data of three orientations of one position on said body surface reference positioning electrode (17) by at least two field sensors preset therein to provide one reference position, which is still with respect to the surface part of said human body.

**Patentansprüche**

1. Feldpositionierungsgerät (1) zum Bestimmen einer Position und Ausrichtung eines Herzkatheters in Bezug auf ein Referenzsystem, umfassend mindestens eine Felderzeugungsvorrichtung (11), die unter der Anregung eines Ansteuersignals unterscheidbare Felder erzeugt; mindestens einen Feldsensor (12, 12', 1002, 1002'), der im Herzkatheter platziert ist, zum Detektieren der unterscheidbaren Felder, um ein Sensorsignal zu erzeugen; eine Positionierungssignalverarbeitungsvorrichtung (13) zum Übertragen des Ansteuerungssignals an die Felderzeugungsvorrichtung (11) und Detektieren einer Ausgabe des Feldsensorsignals, um Daten von fünfdimensionalen Positions- und Ausrichtungskoordinaten der Position am Herzkatheter zu erzeugen, wo der Sensor (12, 12', 1002, 1002') platziert ist, wobei jede Gruppe von erhaltenen Daten von Positionskoordinaten für einen Abweichungsbetrag kalibriert ist, bevor sie dazu verwendet wird, eine Innenwand eines Hohlraums zu simulieren, um den Fehler zu beseitigen, der durch eine Entfernung zwischen der Stelle, an welcher der Sensor platziert ist, und der Spitze des Herzkatheters hervorgerufen wird.

2. Feldpositionierungsgerät nach Anspruch 1, wobei der Herzkatheter des Geräts mehr als einen des Feldsensors umfasst und der mehr als eine Sensor untereinander durch eine gleiche oder ungleiche Entfernung beabstandet ist.

3. Feldpositionierungsgerät nach Anspruch 1 oder 2, wobei die Vielzahl von unterscheidbaren Feldern, die durch die Felderzeugungsvorrichtung erzeugt wird, jeweils unterschiedliche Frequenzen oder unterschiedliche Phasen aufweist oder sowohl unterschiedliche Frequenzen als auch unterschiedliche Phasen aufweist, wobei es sich bei den Feldern um kontinuierliche Wechselstrom-Magnetfelder oder periodisch variierte Gleichstromimpuls-Magnetfelder handelt.

4. Feldpositionierungsgerät nach Anspruch 3, wobei die Positionierungssignalverarbeitungsvorrichtung das Ansteuerungssignal mit dem Sensorsignal kreuzkorreliert.

5. Feldpositionierungsgerät nach Anspruch 1 oder 2, ferner umfassend einen Drucksensor (1008) zum Messen, wie gut ein Kontakt zwischen dem Herzkatheter und der Innenwand eines menschlichen Hohlraums ist, oder ferner umfassend einen Temperatursensor (1007) zum Messen einer Temperatur einer Stelle, an welcher der Temperatursensor (1007) platziert ist, um die Freisetzung elektrischer Energie basierend auf einem Temperatursignal zu steuern.

6. Feldpositionierungsgerät nach Anspruch 1 oder 2, wobei der Herzkatheter ferner eine Elektrode zum Erlangen eines elektrischen Signals der Innenwand des Herzhohlraums der Position umfasst, an welcher der Herzkatheter platziert ist.

**7.** Feldpositionierungsgerät nach Anspruch 6, ferner umfassend:
eine Verarbeitungsvorrichtung für ein elektrisches Signal zum Verarbeiten des elektrischen Signals, das durch die Elektrode erlangt wurde, sodass das elektrische Signal mit Daten der dreidimensionalen Positionskoordinaten und/oder Daten von zwei Ausrichtungskoordinaten in den Daten der fünfdimensionalen Positions- und Ausrichtungskoordinaten einer Vielzahl von Punkten kombiniert wird, die durch die Positionierungssignalverarbeitungsvorrichtung gesammelt wird, um eine Karte auszubilden, welche die Übertragungssituation des elektrischen Signals über Gewebe der Innenwand des Herzhohlraums angibt.

**8.** Dreidimensionales Kartierungsverfahren für eine Innenwand eines menschlichen Hohlraums zum Bestimmen einer Position und Ausrichtung eines Herzkatheters in Bezug auf ein Referenzsystem, wobei das Verfahren die folgenden Schritte umfasst: (a) Erzeugen einer Vielzahl von unterscheidbaren Feldern unter Verwendung eines Ansteuerungssignals; (b) Detektieren der unterscheidbaren Felder durch mindestens einen Sensor, der im Herzkatheter voreingestellt ist, um ein Sensorsignal zu erzeugen; (c) Detektieren einer Ausgabe des Sensorsignals, um Daten von fünfdimensionalen Positions- und Ausrichtungskoordinaten der Position des Herzkatheters zu erlangen, wo der Sensor platziert ist, wobei die Daten von fünfdimensionalen Positions- und Ausrichtungskoordinaten Daten von dreidimensionalen Positionskoordinaten und Daten von zwei Ausrichtungskoordinaten enthalten, wobei jede Gruppe von erhaltenen Daten von Positionskoordinaten für einen Abweichungsbetrag kalibriert ist, bevor sie dazu verwendet wird, eine Innenwand eines Hohlraums zu simulieren, um den Fehler zu beseitigen, der durch eine Entfernung zwischen der Stelle, an welcher der Sensor platziert ist, und der Spitze des Herzkatheters hervorgerufen wird.

**9.** Verfahren nach Anspruch 8, wobei der Herzkatheter mit mehr als einem des Sensors voreingestellt ist, durch welchen das Verfahren ferner die folgenden Schritte umfasst:

(g) Erlangen von fünfdimensionalen Positions- und Ausrichtungskoordinaten der Position am Herzkatheter, wo der mehr als eine Sensor platziert ist, unter Verwendung der Daten, die jeweils durch den mehr als einen Sensor gemessen wurden, der in dem Herzkatheter voreingestellt ist; und
(h) Erlangen eines partiellen oder vollständigen dreidimensionalen Bildmodells des Herzkatheters durch Verarbeiten der erlangten Daten der fünfdimensionalen Positions- und Ausrichtungskoordinaten der Position am Herzkatheter, wo der mehr als eine Sensor platziert ist.

**10.** Verfahren nach Anspruch 9, wobei der mehr als eine Sensor, der in dem Herzkatheter voreingestellt ist, untereinander durch eine gleiche oder ungleiche Entfernung beabstandet ist.

**11.** Verfahren nach Anspruch 10, wobei der Herzkatheter mit zwei Sensoren voreingestellt ist, die durch eine bestimmte Entfernung untereinander beabstandet sind.

**12.** Verfahren nach einem der Ansprüche 8 bis 11, ferner den folgenden Schritt umfassend:
Erlangen eines elektrischen Signals des menschlichen Hohlraums der Position, wo der Herzkatheter platziert ist, unter Verwendung einer Elektrode, die in dem Herzkatheter voreingestellt ist.

**13.** Verfahren nach Anspruch 12, ferner den folgenden Schritt umfassend:
Ausbilden, gemäß einer Zeitabfolge des Erlangens des elektrischen Signals, einer Karte, welche die Übertragungssituation des elektrischen Signals über Gewebe der Innenwand des menschlichen Hohlraums angibt, durch Kombinieren der gesammelten Vielzahl der elektrischen Signale der Innenwand des menschlichen Hohlraums mit den Daten der dreidimensionalen Positionen in den entsprechend gesammelten fünfdimensionalen Daten und Verarbeiten davon.

**14.** Feldpositionierungsgerät nach Anspruch 1, ferner umfassend:
eine Körperoberflächenreferenzpositionierungselektrode (17), die auf einem Oberflächenabschnitt eines menschlichen Körpers montiert ist, zum Bestimmen von Daten von dreidimensionalen Positionen und Daten von drei Ausrichtungen einer Position an der Körperoberflächenreferenzpositionierungselektrode (17) durch mindestens zwei Feldsensoren, die darin voreingestellt sind, um eine Referenzposition bereitzustellen, die in Bezug auf den Oberflächenabschnitt des menschlichen Körpers unbewegt ist.

**Revendications**

**1.** Equipement de positionnement de champ (1) destiné à déterminer une position et une orientation d'un cathéter cardiaque par rapport à un système de référence, comprenant au moins un dispositif générateur de champ (11), qui, sous l'excitation d'un signal d'entraînement, génère des champs distinguables ; au moins un capteur de champ (12, 12', 1002, 1002') situé dans ledit cathéter cardiaque pour détecter les champs distinguables en vue de générer un signal de capteur ; un dispositif de traitement de signal de positionnement (13) destiné à transmettre le signal d'entraînement audit dispositif générateur de champ (11) et à détecter une sortie dudit signal de capteur de champ de manière à générer des données de coordonnées de position et orientation à cinq dimensions de la position sur ledit cathéter cardiaque où est situé le capteur (12, 12', 1002, 1002'), chaque groupe de données obtenues de coordonnées de position est étalonné en termes de quantité de décalage avant d'être utilisé pour simuler une paroi interne de cavité de manière à éliminer l'erreur provoquée par une distance entre l'endroit où est situé le capteur et l'embout dudit cathéter cardiaque.

2. Equipement de positionnement de champ selon la revendication 1, dans lequel ledit cathéter cardiaque de l'équipement comprend plus d'un dit capteur de champ, et ledit plus d'un capteur est mutuellement espacé d'une distance égale ou inégale.

3. Equipement de positionnement de champ selon la revendication 1 ou 2, dans lequel la pluralité de champs distinguables générés par ledit dispositif générateur de champ ont respectivement des fréquences différentes ou des phases différentes, ou ont à la fois des fréquences différentes et des phases différentes, dans lequel lesdits champs sont des champs magnétiques continus à courant alternatif ou des champs magnétiques impulsionnels à courant continu à variation périodique.

4. Equipement de positionnement de champ selon la revendication 3, dans lequel ledit dispositif de traitement de signal de positionnement opère une corrélation croisée entre ledit signal d'entraînement et ledit signal de capteur.

5. Equipement de positionnement de champ selon la revendication 1 ou 2, comprenant en outre un capteur de pression (1008) destiné à mesurer avec quelle qualité un contact entre ledit cathéter cardiaque et ladite paroi interne de cavité humaine et/ou comprend en outre un capteur de température (1007) destiné à mesurer une température d'un endroit où est situé le capteur de température (1007) de manière à commander la libération d'énergie électrique d'après un signal de température.

6. Equipement de positionnement de champ selon la revendication 1 ou 2, dans lequel ledit cathéter cardiaque comprend en outre une électrode destinée à acquérir un signal électrique sur la paroi interne de cavité cardiaque de la position où est situé ledit cathéter cardiaque.

7. Equipement de positionnement de champ selon la revendication 6, comprenant en outre :
un dispositif de traitement de signal électrique destiné à traiter le signal électrique acquis par ladite électrode de telle sorte que ledit signal électrique est combiné avec des données de coordonnées de position en trois dimensions et/ou des données de coordonnées à deux orientations dans les données de coordonnées de position et d'orientation à cinq dimensions d'une pluralité de points collectés par le dispositif de traitement de signal de positionnement pour former une carte indiquant la situation de transmission du signal électrique via un tissu de la paroi interne de cavité cardiaque.

8. Procédé de cartographie tridimensionnelle de paroi interne de cavité humaine destiné à déterminer une position et une orientation d'un cathéter cardiaque par rapport à un système de référence, ledit procédé comprenant des étapes de (a) génération d'une pluralité de champs distinguables par l'utilisation d'un signal d'entraînement ; (b) détection des champs distinguables par au moins un capteur préétabli dans ledit cathéter cardiaque de manière à générer un signal de capteur ; (c) détection d'une sortie du signal de capteur pour acquérir des données de coordonnées de position et orientation à cinq dimensions de la position dudit cathéter cardiaque où est situé le capteur, les données obtenues de coordonnées de position et d'orientation à cinq dimensions contiennent des données de coordonnées de position à trois dimensions, et des données de coordonnées à deux orientations, chaque groupe de données obtenues de coordonnées de position est étalonné en termes de quantité de décalage avant d'être utilisé pour simuler une paroi interne de cavité de manière à éliminer l'erreur provoquée par une distance entre l'endroit où est situé le capteur et l'embout dudit cathéter cardiaque.

9. Procédé selon la revendication 8, dans lequel ledit cathéter cardiaque est préétabli avec plus d'un dit capteur de champ, au moyen duquel le procédé comprend en outre des étapes de :

(g) acquisition de données de coordonnées de position et d'orientation à cinq dimensions de la position du cathéter cardiaque où est situé le plus d'un capteur à l'aide des données respectivement mesurées par le plus d'un capteur préétabli dans ledit cathéter cardiaque ; et

(h) acquisition d'un modèle d'image tridimensionnelle partiel ou complet du cathéter cardiaque par traitement des données acquises de coordonnées de position et orientation à cinq dimensions de la position du cathéter cardiaque où est situé le plus d'un capteur.

10. Procédé selon la revendication 9, dans lequel le plus d'un dit capteur préétabli dans ledit cathéter cardiaque est mutuellement espacé d'une distance égale ou inégale.

11. Procédé selon la revendication 10, dans lequel ledit cathéter cardiaque est préétabli avec deux capteurs qui sont mutuellement espacés d'une certaine distance.

12. Procédé selon l'une quelconque des revendications 8 à 11, comprenant en outre l'étape de :
acquisition d'un signal électrique de la cavité humaine de la position où ledit cathéter cardiaque est situé par l'utilisation d'une électrode préétablie dans ledit cathéter cardiaque.

13. Procédé selon la revendication 12, comprenant en outre l'étape de :
formation, sur une séquence temporelle d'acquisition dudit signal électrique, d'une carte indiquant la situation de transmission du signal électrique via un tissu de la paroi interne de cavité humaine par combinaison de la pluralité collectée desdits signaux électriques de la paroi interne de cavité humaine avec les données des positions en trois dimensions dans les données en cinq dimensions collectées en correspondance et leur traitement.

14. Equipement de positionnement de champ selon la revendication 1, comprenant en outre :
une électrode de positionnement de référence de surface corporelle (17) montée sur une partie de surface d'un corps humain, destinée à déterminer des données de position à trois dimensions et des données de trois orientations d'une position sur ladite électrode de positionnement de référence de surface corporelle (17) par au moins deux capteurs de champ préétablis à l'intérieur pour fournir une position de référence, qui est stationnaire par rapport à la partie de surface dudit corps humain.

Fig.1

```
                    ┌─────────────┐
                    │    Start    │
                    └──────┬──────┘
                           │
                           ▼
        ┌──────────────────────────────────┐        ┌ S101
        │      generating a plurality of    │      ∫
        │      distinguishable fields        │
        └──────────────────┬───────────────┘
                           │
                           ▼
        ┌──────────────────────────────────┐        ┌ S102
        │   detecting the fields to generate a │    ∫
        │  sensor signal with the sensor preset │
        │         in the target device        │
        └──────────────────┬───────────────┘
                           │
                           ▼
        ┌──────────────────────────────────┐        ┌ S103
        │     detecting the sensor signal to  │     ∫
        │    acquire data of five-dimensional │
        │  position and orientation coordinates │
        └──────────────────┬───────────────┘
                           │
                           ▼
    ┌──────────────────────────────────────┐         ┌ S104
    │ acquiring again the data of five-dimensional │  ∫
    │  position and orientation coordinates after │
    │            moving the target device       │
    └──────────────────┬───────────────────┘
                           │
                           ▼
    ┌──────────────────────────────────────┐         ┌ S105
    │ creating a three-dimensional image model by │   ∫
    │  using the acquired data of five-dimensional │
    │     position and orientation coordinates   │
    └──────────────────┬───────────────────┘
                           │
                           ▼
          ◇──────────────────────────────◇           ┌ S106
  No     ◇  Whether does the image satisfy the  ◇    ∫
 ◄───────◇         requirement?              ◇
          ◇──────────────────────────────◇
                           │ Yes
                           ▼
                    ┌─────────────┐
                    │     End     │
                    └─────────────┘
```

Fig.2

Fig.3

Fig.4

Fig.5

Three-dimensional human cavity
electroanotomical mapping system 1''

Surface ECG electrode 18

Electrical signal processing device 19

Workstation 14

Display device 15

Field sensor 12

Body surface reference
positioning electrode 17

Positioning signal processing device 13

Data output device 16

Field generating device 11

Fig.6

Fig.7

Fig.8

Fig.9

Fig.10

Fig.11

Fig.12

**REFERENCES CITED IN THE DESCRIPTION**

**Patent documents cited in the description**

- CN 200910047713
- US 20040254437 A
- CN 1Q1147676 A
- CN 200970234 Y
- CN 201033076 Y
- US 6385482 B
- US 6553326 B
- US 6625563 B
- CN 1168625 A, BEN-HAIM S
- EP 1321097 A2
- WO 2007061890 A2
- EP 1760661 A2